# EUROPEAN PATENT APPLICATION

(11) **EP 4 620 420 A1**
(43) Date of publication of application: **24.09.2025**
(21) Application number: 25162337.7
(22) Date of filing: 07.03.2025
(51) Int. Cl.: A61B 34/20, G06T 7/00, A61B 1/00

(54) **INFORMATION PROCESSING APPARATUS AND INFORMATION PROCESSING PROGRAM**

(30) Priority: 18.03.2024 JP 2024042842
(71) Applicant: FUJIFILM Corporation, Tokyo 106-8620 (JP)
(72) Inventor: CHIBA, Haruto, Tokyo, 106-8620 (JP)
(74) Representative: Dehns Germany Partnerschaft mbB

(57) **Abstract**

Tracking of a viewpoint of an endoscope is autonomously stopped based on an estimation result of the viewpoint of the endoscope.

An information processing apparatus estimates whether or not a viewpoint of an endoscope is trackable from an endoscopic image, tracks the viewpoint of the endoscope using a virtual endoscopic image and a pseudo virtual endoscopic image generated from the endoscopic image in a case where it is estimated that the viewpoint of the endoscope is trackable, determining a tracking result of the viewpoint of the endoscope using the endoscopic image, and a tracking destination virtual endoscopic image and a tracking destination virtual depth image generated from a bronchial model based on the tracked viewpoint of the endoscope, and stops the tracking of the viewpoint of the endoscope until a tracking start instruction is notified in a case where it is determined that the tracking has failed.

## Description

### BACKGROUND OF THE INVENTION

### 1. Field of the Invention

The present invention relates to an information processing apparatus and an information processing program.

### 2. Description of the Related Art

WO2014/141968A discloses an endoscope system that performs image matching, using an endoscopic image and a virtual endoscopic image generated from three-dimensional image data, to estimate a position of an endoscope.

WO2007/129493A discloses a medical image observation support device that acquires an endoscopic image, generates a virtual image from three-dimensional image data, compares bifurcation portion feature information in the endoscopic image with bifurcation portion feature information in the virtual image to associate the endoscopic image with the virtual image, and estimates a position of an endoscope in a case where the endoscopic image is captured.

JP2012-165838A discloses an endoscope insertion support device that acquires an endoscopic image and three-dimensional image data, generates a virtual endoscopic image from the three-dimensional image data, determines a similarity between the endoscopic image and the virtual endoscopic image, and updates position and posture information of a virtual endoscope such that the virtual endoscopic image and the endoscopic image are matched with each other in a case where the endoscopic image and the virtual endoscopic image are not similar to each other.

WO2016/009701A discloses a navigation system that determines whether or not a bifurcation line of a luminal organ is observed, determines whether or not a position of a target bifurcation line is closer to a distal end than a current position of a viewpoint in a case where it is determined that the target bifurcation line has not been observed, and waits until the target bifurcation line is observed in a case where it is determined that the position of the target bifurcation line is closer to the distal end than the current position of the viewpoint.

### SUMMARY OF THE INVENTION

In endoscopy or the like, an information processing apparatus may be used that estimates a viewpoint of an endoscope in a living body from an endoscopic image and notifies a medical worker of a traveling direction of the endoscope such that the endoscope reaches a target location.

For example, in a case where an unknown structure region, which is a region that makes it difficult to specify the shape or pattern of an object, is included in the endoscopic image, information obtained from the endoscopic image may be reduced, and the accuracy of estimation of the viewpoint of the endoscope may be reduced, as compared to a case where the unknown structure region is absent.

In this case, in a case where the traveling direction of the endoscope is determined using the estimated viewpoint of the endoscope without any change, a situation in which the medical worker is notified of an incorrect traveling direction may occur.

The present disclosure has been made in view of the above circumstances, and an object of the present disclosure is to provide an information processing apparatus and an information processing program that can autonomously stop tracking a viewpoint of an endoscope based on an estimation result of the viewpoint of the endoscope.

According to a first aspect of the technology of the present disclosure, there is provided an information processing apparatus comprising: an estimation unit that estimates whether or not a viewpoint of an endoscope that has captured an endoscopic image of a living body is trackable from the endoscopic image; a tracking unit that tracks the viewpoint of the endoscope using the endoscopic image and an image generated from a three-dimensional model of the living body in a case where the estimation unit estimates that the viewpoint of the endoscope is trackable; a determination unit that determines a tracking result of the viewpoint of the endoscope by the tracking unit using the endoscopic image and a three-dimensional model image generated from the three-dimensional model based on the tracked viewpoint of the endoscope; and a controller that performs control to stop the tracking of the viewpoint of the endoscope until a tracking start instruction is notified in a case where the determination unit determines that the tracking has failed.

According to a second aspect of the technology of the present disclosure, in the information processing apparatus according to the first aspect, the three-dimensional model image may be at least one of a tracking destination virtual endoscopic image of the living body, which is an image in a case where the three-dimensional model is viewed from the tracked viewpoint of the endoscope, or a tracking destination virtual depth image, which is a depth image of the three-dimensional model corresponding to a position where the tracking destination virtual endoscopic image has been obtained, and the determination unit may determine the tracking result of the viewpoint of the endoscope using a determination model that has been subjected to machine learning in advance to output whether or not the tracking of the viewpoint of the endoscope has succeeded from the tracking destination virtual endoscopic image, the tracking destination virtual depth image, and the endoscopic image.

According to a third aspect of the technology of the present disclosure, in the information processing apparatus according to the first aspect, the three-dimensional model image may be at least one of a tracking destination virtual endoscopic image of the living body, which is an image in a case where the three-dimensional model is viewed from the tracked viewpoint of the endoscope, or a tracking destination virtual depth image, which is a depth image of the three-dimensional model corresponding to a position where the tracking destination virtual endoscopic image has been obtained, and the determination unit may compare structure information items, which indicate a structure of the living body and have been obtained from the endoscopic image and at least one of the tracking destination virtual endoscopic image or the tracking destination virtual depth image, to determine the tracking result of the viewpoint of the endoscope.

According to a fourth aspect of the technology of the present disclosure, in the information processing apparatus according to any one of the first to third aspects, in a case where the estimation unit estimates that the viewpoint of the endoscope is not trackable from the endoscopic image, the estimation unit may temporarily stop the tracking of the viewpoint of the endoscope until the endoscopic image, from which it is estimated that the viewpoint of the endoscope is trackable, is acquired.

According to a fifth aspect of the technology of the present disclosure, in the information processing apparatus according to any one of the first to third aspects, in a case where the determination unit determines that the tracking has failed, the controller may perform control to display, on a display device, a virtual endoscopic image that is referred to by a medical worker to position the endoscope and that is obtained in a case where the three-dimensional model is viewed from a viewpoint at which the tracking has succeeded.

According to a sixth aspect of the technology of the present disclosure, in the information processing apparatus according to the fifth aspect, the controller may perform control to display, on the display device, the virtual endoscopic image at a viewpoint at which the tracking of the viewpoint of the endoscope has succeeded last before the tracking fails.

According to a seventh aspect of the technology of the present disclosure, in the information processing apparatus according to the fifth aspect, the controller may perform control to display, on the display device, the virtual endoscopic image including a bronchial bifurcation located closer to a viewpoint at which the tracking of the viewpoint of the endoscope has succeeded last before the tracking fails.

According to an eighth aspect of the technology of the present disclosure, in the information processing apparatus according to the fourth aspect, the controller may perform control to notify whether the tracking of the viewpoint of the endoscope is in progress, is temporarily stopped, or is stopped.

According to a ninth aspect of the technology of the present disclosure, in the information processing apparatus according to the eighth aspect, the controller may change a display form of the three-dimensional model image to notify of a tracking state of the viewpoint of the endoscope.

According to a tenth aspect of the technology of the present disclosure, in the information processing apparatus according to the ninth aspect, in a case where the tracking of the viewpoint of the endoscope is temporarily stopped, the controller may perform control to notify of a reason for temporarily stopping the tracking of the viewpoint of the endoscope.

According to an eleventh aspect of the technology of the present disclosure, there is provided an information processing program causing a computer to execute a process comprising: estimating whether or not a viewpoint of an endoscope that has captured an endoscopic image of a living body is trackable from the endoscopic image; tracking the viewpoint of the endoscope using the endoscopic image and an image generated from a three-dimensional model of the living body in a case where it is estimated that the viewpoint of the endoscope is trackable; determining a tracking result of the viewpoint of the endoscope using the endoscopic image and a three-dimensional model image generated from the three-dimensional model based on the tracked viewpoint of the endoscope; and stopping the tracking of the viewpoint of the endoscope until a tracking start instruction is notified in a case where it is determined that the tracking has failed.

According to the present disclosure, it is possible to autonomously stop tracking a viewpoint of an endoscope based on an estimation result of the viewpoint of the endoscope.

### BRIEF DESCRIPTION OF THE DRAWINGS

Fig. 1 is a diagram showing an example of a configuration of an information processing system.
Fig. 2 is a diagram showing an example of an endoscopic image.
Fig. 3 is a diagram showing an example of an endoscopic image including an unknown structure region.
Fig. 4 is a diagram showing an example of machine learning of a trackability estimation model.
Fig. 5 shows an example of a bronchial model.
Fig. 6 is a diagram showing an example of a virtual endoscopic image.
Fig. 7 is a diagram showing an example of a method for estimating a viewpoint difference between images.
Fig. 8 is a diagram showing an example of machine learning of a viewpoint difference estimation model.
Fig. 9 is a diagram showing an example of tracking of a viewpoint of an endoscope.
Fig. 10 is a diagram showing an example of a determination method for determining a tracking result of the viewpoint of the endoscope.
Fig. 11 is a diagram showing an example of a configuration of an information processing apparatus configured by a computer.
Fig. 12 is a flowchart showing an example of a flow of a tracking process.
Fig. 13 is a flowchart showing an example of a flow of a viewpoint tracking process.
Fig. 14 is a diagram showing an example of a display form of a tracking destination virtual endoscopic image.
Fig. 15 is a diagram showing an example of a display form of the tracking destination virtual endoscopic image in a case where a tracking state is changed.
Fig. 16 is a diagram showing an example of display of the unknown structure region in the endoscopic image.
Fig. 17 is a flowchart showing a modification example of the flow of the tracking process.
Fig. 18 is a diagram showing a modification example of a tracking result determination model.
Fig. 19 is a diagram showing an example of a method for estimating a similarity between the images.
Fig. 20 is a flowchart showing an example of a flow of a viewpoint tracking process according to a second embodiment.

### DESCRIPTION OF THE PREFERRED EMBODIMENTS

Hereinafter, embodiments of the present disclosure will be described in detail with reference to the drawings. In addition, the same components and the same processes are denoted by the same reference numerals throughout the drawings, and a duplicate description thereof will be omitted. Dimensional ratios in the drawings are exaggerated for convenience of description and may be different from the actual ratios.

### First Embodiment

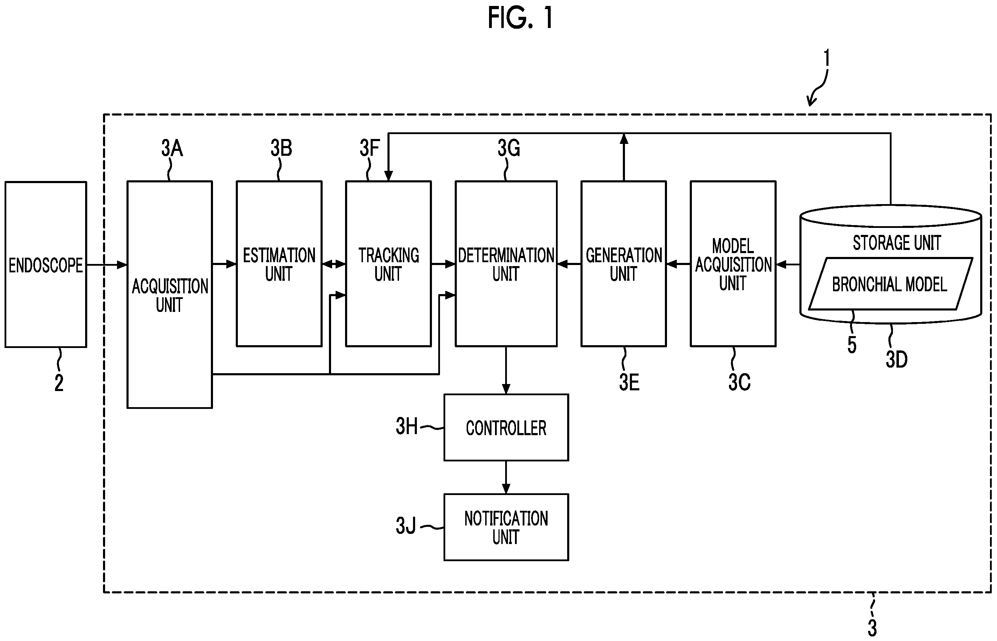

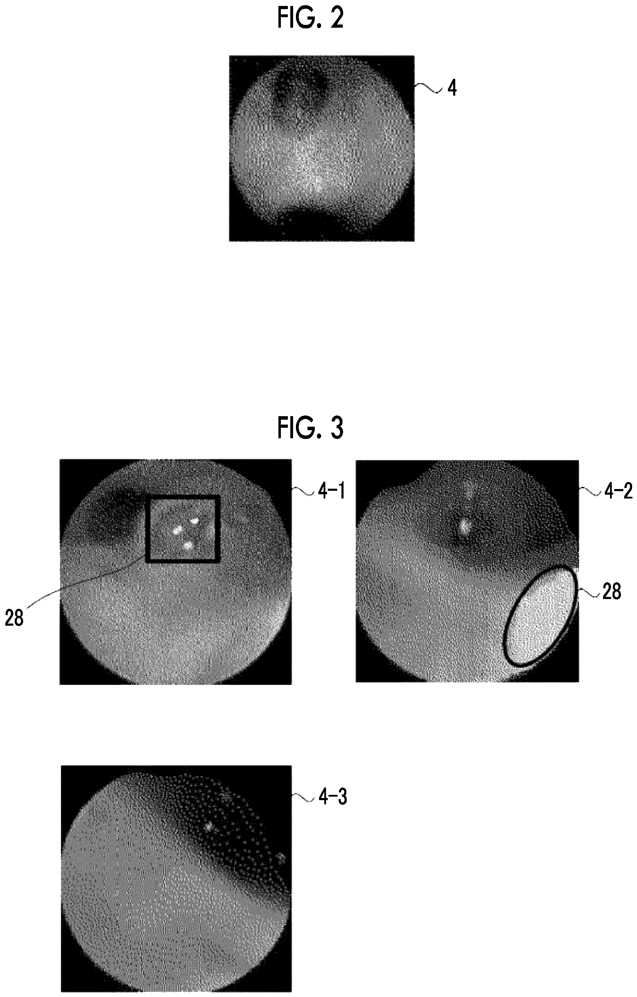

Fig. 1 is a diagram showing an example of a configuration of an information processing system 1 according to a first embodiment. As shown in Fig. 1, the information processing system 1 includes an endoscope 2 and an information processing apparatus 3. An imaging device, such as a camera, is provided at a distal end of the endoscope 2. The information processing system 1 transmits an image captured by the endoscope 2, that is, an endoscopic image 4 to the information processing apparatus 3. Fig. 2 is a diagram showing an example of the endoscopic image 4 captured by the endoscope 2.

There is no restriction on a location of a living body into which the endoscope 2 is inserted, and the endoscope 2 may be, for example, an endoscope 2 for a digestive organ or a ureteroscope. However, as an example, the endoscope 2 according to the embodiment of the present disclosure will be described as a bronchoscope that is inserted into a bronchus of a subject.

In a case where the endoscope 2 is inserted into the bronchus and advanced to a position to be examined, the bronchi have many bifurcations and have a similar shape. Therefore, in some cases, a navigation device is used that notifies a medical worker who operates the endoscope 2 of support information indicating the position and direction of the endoscope 2 in the bronchus and a direction in which the endoscope 2 is to be advanced to reach the position to be examined (hereinafter, referred to as a "target location").

The information processing apparatus 3 is an example of the navigation device that notifies the medical worker of a traveling direction of the endoscope 2. The information processing apparatus 3 includes functional units of an acquisition unit 3A, an estimation unit 3B, a model acquisition unit 3C, a storage unit 3D, a generation unit 3E, a tracking unit 3F, a determination unit 3G, a controller 3H, and a notification unit 3J.

The acquisition unit 3A acquires the endoscopic image 4 from the endoscope 2.

An image of the inside of the bronchus of the subject is displayed in the endoscopic image 4. However, the endoscopic image 4 may include an unknown structure region. The unknown structure region means a region of the endoscopic image 4 that is not capable of being used to estimate the position and posture of the endoscope 2 in a case where the information processing apparatus 3 estimates the position and posture of the endoscope 2 using the endoscopic image 4.

Examples of the unknown structure region include a region having bubbles attached thereto, a region in which the endoscope 2 is out of focus, a region in which the image is blocked by a substance attached to a lens of the endoscope 2, a reflection region in which light illumination is reflected, a whiteout region, and a blackout region. That is, the unknown structure region is a general term for a region that makes it difficult to specify an object.

Fig. 3 is a diagram showing an example of the endoscopic image 4 including the unknown structure region. In a region indicated by a display frame 28 of an endoscopic image 4-1 shown in Fig. 3, bubbles attached to the bronchus which is an example of the unknown structure region are recognized. In a region indicated by a display frame 28 of an endoscopic image 4-2, the reflection of illumination light is recognized. Further, an endoscopic image 4-3 is an example of a so-called out-of-focus image in which the endoscope 2 is too close to the bronchus and does not focus on the bronchus.

In a case where the information processing apparatus 3 estimates the position and posture of the endoscope 2 using the endoscopic image 4 including the unknown structure region that does not correctly represent a pattern of a bronchial wall (referred to as a "texture of a bronchus") due to the shape of the bronchus, the state of blood vessels and tissues, and the like, there is a concern that the accuracy of estimation will be reduced, as compared to a case where the endoscopic image 4 in which the unknown structure region is not present is used.

Therefore, the estimation unit 3B estimates whether or not it is possible to track the position and posture of the endoscope 2 which has captured the endoscopic image 4 from the endoscopic image 4 based on attribute information of the unknown structure region, such as the presence or absence, type, and range of the unknown structure region included in the endoscopic image 4. That is, in a case where the estimation unit 3B estimates the position and posture of the endoscope 2 from the endoscopic image 4, the estimation unit 3B estimates whether or not an error of the estimated position and posture of the endoscope 2 with respect to the actual position and posture of the endoscope 2 is included within an allowable range.

In addition, the posture of the endoscope 2 is a direction in which the endoscope 2 is facing. Since an imaging range of the endoscopic image 4 captured by the endoscope 2 is determined by the position and posture of the endoscope 2, hereinafter, the position and posture of the endoscope 2 is referred to as a "viewpoint of the endoscope 2". In addition, whether or not the viewpoint of the endoscope 2 can be tracked is represented using the expression "trackability of the endoscope 2".

The trackability of the viewpoint of the endoscope 2 is determined by, for example, a score that is output from a trackability estimation model 21 in a case where the endoscopic image 4 is input to the trackability estimation model 21. The score is a value representing the degree of trackability of the endoscope 2. For example, as the score is higher, the probability that the viewpoint of the endoscope 2 will be tracked from the input endoscopic image 4 is higher. In the present disclosure, for example, it is assumed that the score represents the probability that the viewpoint of the endoscope 2 will be tracked.

The trackability estimation model 21 is, for example, a model generated in advance by machine learning and is stored in the storage unit 3D in advance. The information processing apparatus 3 shown in Fig. 1 includes the storage unit 3D. However, the information processing apparatus 3 does not necessarily include the storage unit 3D. For example, an external apparatus other than the information processing apparatus 3, such as a data server, may be used as the storage unit 3D.

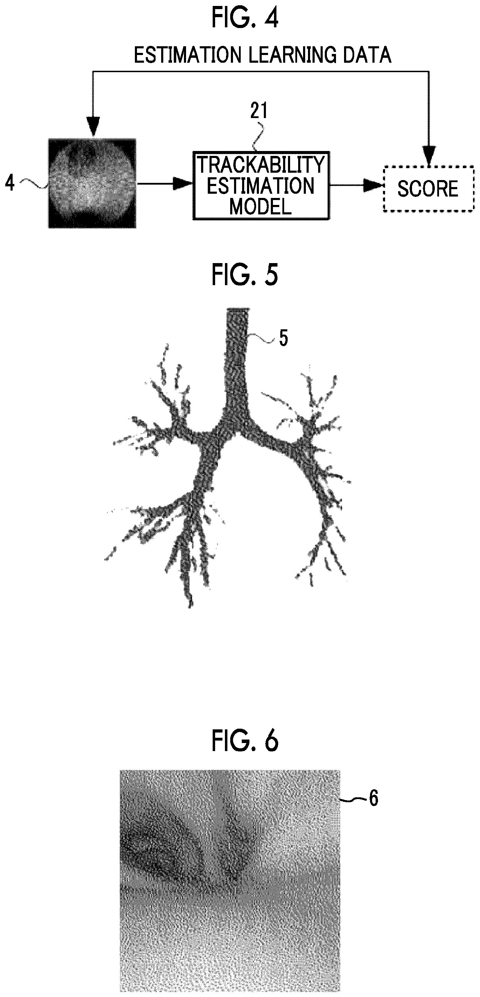

Fig. 4 is a diagram showing an example of the machine learning of the trackability estimation model 21. For example, the medical worker prepares a plurality of learning data items (hereinafter, referred to as "estimation learning data items") in advance in which the score is associated with each of the endoscopic images 4 captured in advance. The medical worker associates the score with each endoscopic image 4 based on the state of the unknown structure region, such as the presence or absence, type, and range of the unknown structure region in the endoscopic image 4.

In addition, in a case where the endoscopic image 4 included in the estimation learning data is input, machine learning is performed on the trackability estimation model 21 such that the score associated with the input endoscopic image 4 is output from the trackability estimation model 21. That is, in the estimation learning data, the endoscopic image 4 is input data, and the score is training data.

For example, a convolutional neural network (CNN) or the like is used as the trackability estimation model 21. For example, deep learning is used as a machine learning method.

In addition, the image used by a CPU 10A to estimate the trackability of the endoscope 2 is not limited to the endoscopic image 4. The CPU 10A may estimate the trackability of the endoscope 2 using the image generated from the endoscopic image 4 or another image other than the endoscopic image 4 in which the shape of the bronchus can be recognized. In addition, the CPU 10A may combine a plurality of types of images to estimate the trackability of the endoscope 2.

For example, the CPU 10A may estimate the trackability of the endoscope 2 using a depth image corresponding to the endoscopic image 4. The depth image corresponding to the endoscopic image 4 is, for example, an image in which the shape of the bronchus at a position where the distal end of the endoscope 2 is present is represented as distance data from an entrance of the bronchus using distance data obtained from a depth sensor that measures the distance from the entrance of the bronchus. For example, a stereo camera, a time-of-flight (TOF) sensor, a light detection and ranging (LiDAR) sensor, or a photoelectric sensor is used as the depth sensor. In addition, the CPU 10A may combine the endoscopic image 4 with the depth image to estimate the trackability of the endoscope 2 or may estimate the trackability of the endoscope 2 using positional information of the unknown structure region or brightness information of the endoscopic image 4.

In this case, machine learning may be performed on the trackability estimation model 21, using the estimation learning data in which the image used for estimating the trackability of the endoscope 2 is used as input data and the score is used as training data.

The estimation unit 3B shown in Fig. 1 compares the score output from the trackability estimation model 21 with a predetermined reference score. The reference score is a value indicating a minimum score at which an estimation error of the viewpoint of the endoscope 2 is considered to be included within an allowable range in a case where the viewpoint of the endoscope 2 is tracked, is set in advance by, for example, the medical worker, and is stored in the storage unit 3D in advance.

In a case where the score is less than the reference score, there is a high probability that the tracked viewpoint of the endoscope 2 will be incorrect even though the viewpoint of the endoscope 2 is tracked from the endoscopic image 4 acquired by the acquisition unit 3A. Therefore, the estimation unit 3B does not request the tracking unit 3F to track the viewpoint of the endoscope 2 until the acquisition unit 3A acquires the endoscopic image 4, in which it is estimated that the viewpoint of the endoscope 2 can be tracked, thereby temporarily stopping the tracking of the viewpoint of the endoscope 2. On the other hand, in a case where the score is equal to or greater than the reference score, the estimation unit 3B requests the tracking unit 3F to track the viewpoint of the endoscope 2.

The tracking unit 3F that has received the request to track the viewpoint of the endoscope 2 from the estimation unit 3B tracks the viewpoint of the endoscope 2 using the endoscopic image 4 and an image generated from a three-dimensional model of the living body of the subject.

Specifically, the tracking unit 3F estimates a viewpoint difference between the endoscopic image 4 and a virtual endoscopic image 6 generated from a bronchial model 5 of the subject which will be described below (simply, may be referred to as a "viewpoint difference between images"). The tracking unit 3F tracks the viewpoint of the endoscope 2 using the estimated viewpoint difference between the images. That is, the tracking unit 3F tracks the viewpoint of the endoscope 2 in a case where the score is equal to or greater than the reference score. In addition, a method for estimating the viewpoint difference between the images in the tracking unit 3F will be described in detail below.

Meanwhile, the bronchial model 5 representing the shape of the bronchus of the subject is used to notify the medical worker of the traveling direction of the endoscope 2. The bronchial model 5 is an example of a three-dimensional model that is generated in advance by, for example, computed tomography (CT) before endoscopy. Fig. 5 shows an example of the bronchial model 5. The bronchial model 5 is generated in advance for each subject and is stored in advance in the storage unit 3D.

The model acquisition unit 3C shown in Fig. 1 acquires the bronchial model 5 of the subject to be examined from the storage unit 3D.

The generation unit 3E generates an image in a case where the bronchial model 5 is viewed from a predetermined position inside the bronchial model 5, that is, the virtual endoscopic image 6, using the bronchial model 5 acquired by the model acquisition unit 3C. For example, a position where the distal end of the endoscope 2 is estimated to be present is used as the predetermined position inside the bronchial model 5. Fig. 6 is a diagram showing an example of the virtual endoscopic image 6 generated by the generation unit 3E using the bronchial model 5.

In addition, the virtual endoscopic image 6 is not necessarily a two-dimensional image and may be a three-dimensional image generated using three-dimensional imaging data obtained by CT, magnetic resonance imaging (MRI), or the like.

Since the bronchial model 5 is a three-dimensional model generated from data representing the shape of the bronchus of the subject, bubbles or foreign substances are not attached to the bronchial model 5 unlike the actual bronchus of the subject. In addition, since the virtual endoscopic image 6 is generated by data processing using the bronchial model 5, the virtual endoscopic image 6 is not out of focus, illumination light is not reflected, and whiteout or blackout does not occur. That is, the virtual endoscopic image 6 is an image that faithfully reproduces how the shape of the bronchial model 5 looks in a case where the bronchial model 5 is viewed from the designated viewpoint. Since the viewpoint of the endoscope 2 can be freely set in the bronchial model 5, the generation unit 3E can generate the virtual endoscopic image 6 from any viewpoint.

The tracking unit 3F shown in Fig. 1 estimates a viewpoint difference between the viewpoint at which the endoscopic image 4 has been obtained and the viewpoint at which the virtual endoscopic image 6 has been obtained from the deviation between the shapes of each bronchus or the textures of each bronchus included in the endoscopic image 4, from which the viewpoint of the endoscope 2 is estimated to be trackable, and any one of the virtual endoscopic images 6 generated by the generation unit 3E.

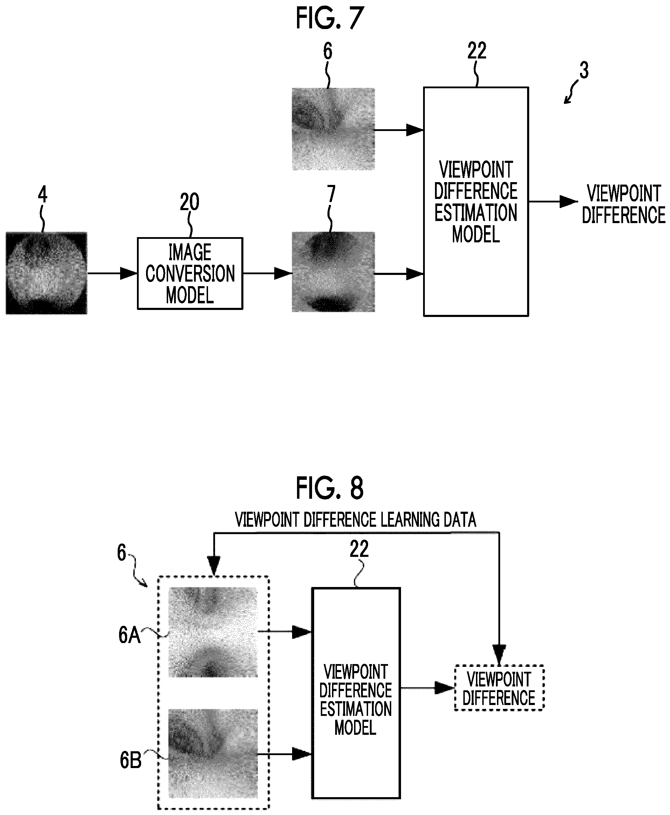

Specifically, a method for estimating the viewpoint difference between the images in the tracking unit 3F will be described. Fig. 7 is a diagram showing an example of the method for estimating the viewpoint difference between the images. The tracking unit 3F estimates the viewpoint difference between the images using a viewpoint difference estimation model 22 that has been subjected to machine learning in advance to output the viewpoint difference between the endoscopic image 4 and the virtual endoscopic image 6 for the endoscopic image 4 and the virtual endoscopic image 6 in a case where the bronchial model 5 is viewed from the designated viewpoint. In addition, instead of the endoscopic image 4, an image generated from the endoscopic image 4 may be input to the viewpoint difference estimation model 22. The image generated from the endoscopic image 4 is also an example of the endoscopic image 4.

As can be seen from the fact that the viewpoint difference estimation model 22 outputs the viewpoint difference between the input endoscopic image 4 and the input virtual endoscopic image 6, the virtual endoscopic image 6 input to the viewpoint difference estimation model 22 may be the virtual endoscopic image 6 obtained from a viewpoint different from the viewpoint at which the endoscopic image 4 has been captured.

The tracking unit 3F inputs the endoscopic image 4 to an image conversion model 20 to convert the endoscopic image 4 into a pseudo virtual endoscopic image 7.

The pseudo virtual endoscopic image 7 is an image obtained by converting the endoscopic image 4 into an expression format that looks like the virtual endoscopic image 6. Specifically, the pseudo virtual endoscopic image 7 is an image obtained by removing the texture of the bronchus or the unknown structure region from the endoscopic image 4 such that the shape of the bronchus is easier to ascertain than that in the endoscopic image 4.

The image conversion model 20 is a model generated in advance using, for example, a generative adversarial network (GAN) trained to output the pseudo virtual endoscopic image 7 similar to the virtual endoscopic image 6 from the endoscopic image 4 and is stored in the storage unit 3D in advance.

The tracking unit 3F inputs the virtual endoscopic image 6 and the pseudo virtual endoscopic image 7 obtained as described above to the viewpoint difference estimation model 22 to estimate the viewpoint difference between the endoscopic image 4, which is a generation source of the pseudo virtual endoscopic image 7, and the virtual endoscopic image 6. That is, the pseudo virtual endoscopic image 7 is also an example of the endoscopic image 4 input to the viewpoint difference estimation model 22.

In addition, the reason for using the image obtained by processing the endoscopic image 4 to be close to the virtual endoscopic image 6, such as the pseudo virtual endoscopic image 7, as the input of the viewpoint difference estimation model 22 instead of the endoscopic image 4 is to consider ease of the preparation of learning data (hereinafter, referred to as "viewpoint difference learning data") used for machine learning of the viewpoint difference estimation model 22.

For example, two endoscopic images 4 whose viewpoints deviate from each other and information related to the viewpoint difference between the endoscopic images 4 are used to perform the machine learning of the viewpoint difference estimation model 22. However, the endoscope 2 is being miniaturized such that the endoscope 2 can be inserted into as many bronchi as possible due to the characteristics of the endoscope 2. For example, it is difficult to separately attach sensors for measuring the viewpoint of the endoscope 2, such as a gyro sensor and a motion sensor. In addition, since the endoscope 2 is inserted into the body, it is possible to ascertain the viewpoint of the endoscope 2 only through the endoscopic image 4 with a limited angle of view, and it is also difficult to acquire the viewpoint difference from the endoscopic image 4.

In contrast, as described above, the viewpoint of the endoscope 2 can be freely set in the bronchial model 5. Since the bronchial model 5 is a three-dimensional model based on examination data of CT or the like, a correct viewpoint difference can be acquired by numerical calculation.

Therefore, in a case where a combination of two virtual endoscopic images 6 whose viewpoints deviate from each other and information related to the viewpoint difference between the virtual endoscopic images 6 is used as the viewpoint difference learning data, it is possible to more easily prepare a larger amount of viewpoint difference training data, as compared to a case where a combination of two endoscopic images 4 whose viewpoints deviate from each other and information related to the viewpoint difference between the endoscopic images 4 is used as the viewpoint difference learning data.

For the above reason, two virtual endoscopic images 6 having different viewpoints are used as the input data in the viewpoint difference learning data of the viewpoint difference estimation model 22. Therefore, in a case where the endoscopic image 4 is input to the viewpoint difference estimation model 22 without any change, the accuracy of estimation of the viewpoint difference by the viewpoint difference estimation model 22 may be reduced. Therefore, the process of converting the endoscopic image 4 into the pseudo virtual endoscopic image 7 and then inputting the pseudo virtual endoscopic image 7 to the viewpoint difference estimation model 22 is performed.

Of course, the endoscopic image 4 may be input to the viewpoint difference estimation model 22 without any change. In this case, the machine learning of the viewpoint difference estimation model 22 may be performed using viewpoint difference learning data obtained by combining the endoscopic image 4, the virtual endoscopic image 6 having a viewpoint that deviates from the viewpoint of the endoscopic image 4, and information related to the viewpoint difference between the images.

The viewpoint difference estimation model 22 is a model generated in advance by the machine learning and is stored in the storage unit 3D in advance.

Fig. 8 is a diagram showing an example of the machine learning of the viewpoint difference estimation model 22.

A plurality of viewpoint difference learning data items in which the viewpoint difference between two virtual endoscopic images 6 having different viewpoints is associated with the virtual endoscopic images 6 are prepared in advance. In Fig. 8, a virtual endoscopic image 6A and a virtual endoscopic image 6B indicate the two virtual endoscopic images 6 having different viewpoints.

In addition, the machine learning of the viewpoint difference estimation model 22 is performed such that, in a case where the virtual endoscopic images 6A and 6B included in the viewpoint difference learning data are input, the viewpoint difference estimation model 22 outputs the viewpoint difference associated with each input. For example, a CNN or the like is used as the viewpoint difference estimation model 22. For example, deep learning is used as the machine learning method.

That is, in the viewpoint difference learning data, the virtual endoscopic images 6A and 6B are input data, and the viewpoint difference is training data.

In addition, the machine learning of the viewpoint difference estimation model 22 may be performed using a range of the bronchial model 5 (referred to as a "partial bronchial model") viewed from each viewpoint, instead of any one of the virtual endoscopic image 6A or the virtual endoscopic image 6B. That is, any one of the virtual endoscopic images 6 may be represented as three-dimensional data. In this case, in the method for estimating the viewpoint difference between the images shown in Fig. 7, the partial bronchial model at a designated viewpoint is used instead of the virtual endoscopic image 6.

In addition, supplementary information related to the position of the endoscope 2 may be added as the input data of the viewpoint difference learning data of the viewpoint difference estimation model 22. In this case, the information processing apparatus 3 estimates the viewpoint difference of the endoscope 2 using the endoscopic image 4, the virtual endoscopic image 6, the supplementary information, and the viewpoint difference estimation model 22. For example, the depth image of the bronchial model 5 at the position of the viewpoint at which the virtual endoscopic images 6A and 6B have been obtained is used as the supplementary information.

In a case where the supplementary information is added to the input of the viewpoint difference estimation model 22, the accuracy of estimation of the viewpoint difference may be improved as compared to a case where the viewpoint difference of the endoscope 2 is estimated without using the supplementary information.

Since the virtual endoscopic image 6 is an image generated from the bronchial model 5, the viewpoint of the virtual endoscopic image 6 is known to the tracking unit 3F. Therefore, the tracking unit 3F can specify the viewpoint of the endoscope 2 from the viewpoint of the virtual endoscopic image 6 and the viewpoint difference between the estimated endoscopic image 4 and the virtual endoscopic image 6.

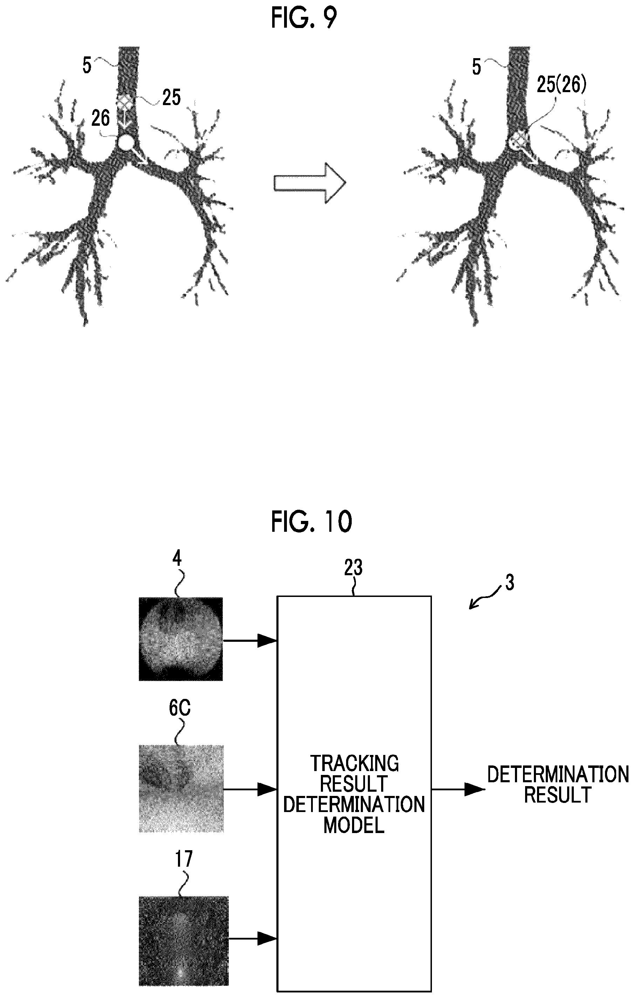

Fig. 9 is a diagram showing an example of the tracking of the viewpoint of the endoscope 2. In Fig. 9, a mark 25 indicates a predetermined position inside the bronchial model 5 (for example, a schematic position where the endoscope 2 is considered to be present), and a mark 26 indicates an actual position of the endoscope 2. The tracking unit 3F can move the position of the mark 25 to the position of the mark 26 from the viewpoint difference between the images and take the same posture as the endoscope 2 whose position is indicated by the mark 26 on the bronchial model 5. In this way, the tracking unit 3F tracks the viewpoint of the endoscope 2 based on the estimated viewpoint difference.

In a case where the viewpoint of the endoscope 2 indicated by the endoscopic image 4 is known, a tracking destination virtual endoscopic image 6C, which is the virtual endoscopic image 6 in a case where the bronchial model 5 is viewed from the tracked viewpoint, can be generated from the bronchial model 5.

The tracking destination virtual endoscopic image 6C is an example of the three-dimensional model image generated from the three-dimensional model based on the tracked viewpoint of the endoscope 2. In addition, since the tracking destination virtual endoscopic image 6C is an image obtained as a tracking result of the viewpoint of the endoscope 2, the tracking destination virtual endoscopic image 6C is also the tracking result of the viewpoint of the endoscope 2. The tracking unit 3F notifies the determination unit 3G of the generated tracking destination virtual endoscopic image 6C.

The determination unit 3G shown in Fig. 1 that has received the tracking destination virtual endoscopic image 6C determines whether or not the endoscopic image 4 and the tracking destination virtual endoscopic image 6C are similar images, using the endoscopic image 4 used by the tracking unit 3F to track the viewpoint of the endoscope 2 and the tracking destination virtual endoscopic image 6C received from the tracking unit 3F.

The fact that the endoscopic image 4 and the tracking destination virtual endoscopic image 6C are similar to each other means that the deviation between the shapes of the bronchus included in the endoscopic image 4 and the tracking destination virtual endoscopic image 6C is within a predetermined allowable range in which the images can be considered to be similar to each other. The fact that the tracking destination virtual endoscopic image 6C is similar to the endoscopic image 4 means that the bronchial model 5 is viewed from the same viewpoint as the current viewpoint of the endoscope 2 and thus indicates that the tracking of the viewpoint of the endoscope 2 has succeeded. On the other hand, the fact that the tracking destination virtual endoscopic image 6C is not similar to the endoscopic image 4 means that the bronchial model 5 is viewed from a viewpoint different from the current viewpoint of the endoscope 2 and thus indicates that the tracking of the viewpoint of the endoscope 2 has failed. That is, the determination unit 3G determines whether or not the tracking unit 3F has succeeded in tracking the viewpoint of the endoscope 2.

Fig. 10 is a diagram showing an example of a determination method for determining the tracking result of the viewpoint of the endoscope 2. The determination unit 3G determines the tracking result of the viewpoint of the endoscope 2, using a tracking result determination model 23 that has been subjected to machine learning in advance to output whether or not the tracking of the viewpoint of the endoscope 2 has succeeded for the endoscopic image 4, the tracking destination virtual endoscopic image 6C, and a tracking destination virtual depth image 17 which is a depth image of the bronchial model 5 corresponding to the position where the tracking destination virtual endoscopic image 6C has been obtained (also referred to as a "depth image corresponding to the position of the viewpoint of the endoscope 2").

The tracking destination virtual depth image 17 is, for example, an image indicating a distance from the entrance of the bronchial model 5 to the vicinity of the viewpoint at which the tracking destination virtual endoscopic image 6C has been generated. That is, the tracking destination virtual depth image 17 is the depth image of the bronchial model 5 corresponding to the position where the tracking destination virtual endoscopic image 6C has been obtained.

As already described above, the tracking result of the viewpoint of the endoscope 2 is determined based on the similarity between the endoscopic image 4 and the tracking destination virtual endoscopic image 6C. Therefore, the tracking destination virtual depth image 17 is not necessarily used to determine the tracking result of the viewpoint of the endoscope 2. However, the tracking destination virtual depth image 17 is supplementary information related to the position of the endoscope 2. Therefore, in a case where the tracking destination virtual depth image 17 is used to determine the tracking result of the viewpoint of the endoscope 2, the accuracy of determination may be improved, as compared to a case where the tracking result of the viewpoint of the endoscope 2 is determined only from the endoscopic image 4 and the tracking destination virtual endoscopic image 6C.

In addition, the tracking result of the viewpoint of the endoscope 2 may be determined based on the endoscopic image 4 and the tracking destination virtual depth image 17. However, in a case where the tracking destination virtual endoscopic image 6C is used to determine the tracking result of the viewpoint of the endoscope 2, the accuracy of determination may be improved as compared to a case where the tracking result of the viewpoint of the endoscope 2 is determined only from the endoscopic image 4 and the tracking destination virtual depth image 17.

Therefore, hereinafter, an example in which the endoscopic image 4, the tracking destination virtual endoscopic image 6C, and the tracking destination virtual depth image 17 are input to the tracking result determination model 23 to determine the tracking result of the viewpoint of the endoscope 2 will be described.

In addition, the tracking result determination model 23 may be a model that outputs the tracking result of the viewpoint of the endoscope 2 in response to the input of the endoscopic image 4 and the tracking destination virtual depth image 17.

The tracking destination virtual endoscopic image 6C and the tracking destination virtual depth image 17 generated from the bronchial model 5 are examples of the three-dimensional model image.

Determination learning data is used for the machine learning of the tracking result determination model 23 that outputs the tracking result of the viewpoint of the endoscope 2 from the endoscopic image 4, the tracking destination virtual endoscopic image 6C, and the tracking destination virtual depth image 17.

A plurality of pair data items in which the endoscopic image 4 captured in advance and the virtual endoscopic image 6 generated from the viewpoint of the bronchial model 5 corresponding to the viewpoint at which the endoscopic image 4 has been captured are associated with each other are prepared in advance in order to generate the determination learning data. The viewpoint at which the endoscopic image 4 has been captured and the viewpoint at which the virtual endoscopic image 6 has been generated in the pair data are not necessarily matched with each other and may be within an allowable range in which the viewpoints can be considered to be the same.

The medical worker randomly shifts the viewpoint of the bronchial model 5 used to generate the virtual endoscopic image 6 included in each prepared pair data item within a first range. The first range indicates a range in which the deviation between the shifted viewpoint after being shifted with respect to the bronchial model 5 and the viewpoint of the endoscope 2 at which the endoscopic image 4 included in the same pair data as the virtual endoscopic image 6 generated from the bronchial model 5 before the shift of the viewpoint has been captured is within an allowable range and in which the viewpoints can be considered to be the same. That is, the first range is a range in which the tracking of the viewpoint of the endoscope 2 can be considered to be successful.

The medical worker generates the virtual endoscopic image 6 obtained in a case where the bronchial model 5 is viewed from the viewpoint after being shifted within the first range, using the bronchial model 5, generates a depth image corresponding to the viewpoint after being shifted within the first range, and associates the virtual endoscopic image 6 and the depth image whose viewpoints have been shifted with the endoscopic image 4 of the pair data which is a generation source of the virtual endoscopic image 6 and the depth image whose viewpoints have been shifted. Further, the medical worker associates a label "tracking success" with the endoscopic image 4 and a combination of the virtual endoscopic image 6 and the depth image generated by shifting the viewpoint within the first range to generate the determination learning data indicating that the tracking of the endoscope 2 has succeeded.

On the other hand, the medical worker randomly shifts the viewpoint in the bronchial model 5 used to generate the virtual endoscopic image 6 included in each prepared pair data item to a range (referred to as a "second range") exceeding the first range. The second range indicates a range in which the deviation between the viewpoint after being shifted with respect to the bronchial model 5 and the viewpoint of the endoscope 2 at which the endoscopic image 4 included in the same pair data as the virtual endoscopic image 6 generated from the bronchial model 5 before the shift of the viewpoint has been captured exceeds the allowable range and in which the viewpoints can be considered to be different. That is, the second range is a range in which the tracking of the viewpoint of the endoscope 2 can be considered to fail.

The medical worker generates the virtual endoscopic image 6 obtained in a case where the bronchial model 5 is viewed from the viewpoint after being shifted to the second range, using the bronchial model 5, generates a depth image corresponding to the viewpoint after being shifted to the second range, and associates the virtual endoscopic image 6 and the depth image whose viewpoints have been shifted with the endoscopic image 4 of the pair data which is a generation source of the virtual endoscopic image 6 and the depth image whose viewpoints have been shifted. Further, the medical worker associates a label "tracking failure" with the endoscopic image 4 and a combination of the virtual endoscopic image 6 and the depth image whose viewpoints have been shifted to the second range to generate the determination learning data indicating that the tracking of the endoscope 2 has failed.

As described above, the determination learning data indicating that the tracking of the endoscope 2 has succeeded and the determination learning data indicating that the tracking of the endoscope 2 has failed are generated and are used for the machine learning of the tracking result determination model 23. In the determination learning data, the endoscopic image 4, the virtual endoscopic image 6, and the depth image are input data, and the labels "tracking success" and "tracking failure" are training data.

Further, in a case where the tracking result determination model 23 is a model that outputs the tracking result of the viewpoint of the endoscope 2 in response to the input of the endoscopic image 4 and the tracking destination virtual depth image 17, the medical worker may generate the determination learning data in which the label "tracking success" or "tracking failure" has been associated with a combination of the endoscopic image 4 and the depth image whose viewpoint has been shifted according to the degree of shift of the viewpoint. In addition, in a case where the tracking result determination model 23 is a model that outputs the tracking result of the viewpoint of the endoscope 2 in response to the input of the endoscopic image 4 and the tracking destination virtual endoscopic image 6C, the medical worker may generate the determination learning data in which the label "tracking success" or "tracking failure" has been associated with a combination of the endoscopic image 4 and the virtual endoscopic image 6 whose viewpoint has been shifted according to the degree of shift of the viewpoint. Needless to say, the endoscopic image 4 may be replaced with a depth image corresponding to the endoscopic image 4.

The determination unit 3G notifies the controller 3H of the tracking result of the viewpoint of the endoscope 2 determined in this way.

In a case where the tracking result of the viewpoint of the endoscope 2 received from the determination unit 3G is "tracking success", the controller 3H shown in Fig. 1 controls other functional units to continue the tracking of the viewpoint of the endoscope 2. In addition, the controller 3H determines a traveling direction in which the endoscope 2 reaches the target location using the bronchial model 5 based on the tracked viewpoint of the endoscope 2 and controls the notification unit 3J such that the notification unit 3J notifies the medical worker of the determined traveling direction of the endoscope 2.

On the other hand, in a case where the tracking result of the viewpoint of the endoscope 2 received from the determination unit 3G is "tracking failure", the controller 3H performs control to stop the tracking of the viewpoint of the endoscope 2 until a tracking start instruction is notified from the medical worker.

In addition, the controller 3H controls the notification unit 3J such that the notification unit 3J notifies the medical worker of the tracking state of the viewpoint of the endoscope 2 such as whether the process of tracking the viewpoint of the endoscope 2 is being executed, is stopped, or is temporarily stopped. Further, the controller 3H controls the notification unit 3J such that various images used for the process of tracking the viewpoint of the endoscope 2, such as the endoscopic image 4 and the tracking destination virtual endoscopic image 6C, or various images generated by the process of tracking the viewpoint of the endoscope 2.

For example, the notification unit 3J displays the tracking state of the viewpoint of the endoscope 2, various images, and the like on the display or notifies the medical worker of the traveling direction of the endoscope 2 by voice under the control of the controller 3H to notify the medical worker of various types of information useful for the navigation of the endoscope 2.

The machine learning of various models, such as the image conversion model 20, the trackability estimation model 21, the viewpoint difference estimation model 22, and the tracking result determination model 23 used for the process of tracking the viewpoint of the endoscope 2, and the generation of the bronchial model 5 are not necessarily performed by the information processing apparatus 3 and may be performed by another computer having a higher processing capacity than the information processing apparatus 3. In this case, the information processing apparatus 3 acquires various models generated by another computer from another computer and uses the various models.

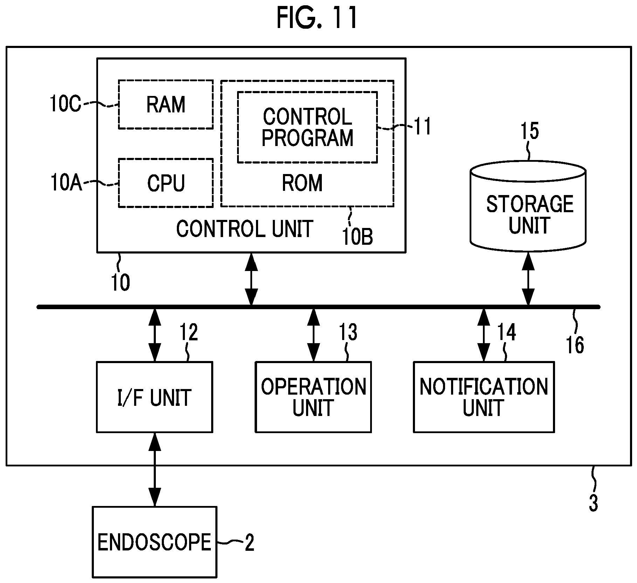

The information processing apparatus 3 shown in Fig. 1 that has these functions is configured by, for example, a computer. Fig. 11 is a diagram showing an example of a configuration of the information processing apparatus 3 configured by the computer.

As shown in Fig. 11, the information processing apparatus 3 comprises a control unit 10, an interface (I/F) unit 12, an operation unit 13, a notification unit 14, and a storage unit 15. The control unit 10, the I/F unit 12, the operation unit 13, the notification unit 14, and the storage unit 15 are connected to each other via a bus 16 such that they can transmit and receive various types of information.

The control unit 10 controls the operation of the information processing apparatus 3 based on an instruction from the medical worker. The control unit 10 is an example of a processor and comprises a central processing unit (CPU) 10A that is in charge of processes of each functional unit of the information processing apparatus 3 shown in Fig. 1, a read only memory (ROM) 10B, and a random access memory (RAM) 10C. The ROM 10B stores in advance various programs including a control program 11 that is read by the CPU 10A to determine the tracking result of the viewpoint of the endoscope 2 and various parameters that are referred to by the CPU 10A to control the operation of the information processing apparatus 3. The RAM 10C is used as a temporary work area of the CPU 10A.

The I/F unit 12 transmits and receives various types of information to and from the endoscope 2 using wireless communication or wired communication. The information processing apparatus 3 acquires the endoscopic image 4 from the endoscope 2 via the I/F unit 12.

The operation unit 13 is used, for example, by the medical worker to input an instruction related to the tracking of the viewpoint of the endoscope 2, various types of information, and the like. There is no restriction on the operation form of the operation unit 13. For example, it is possible to receive the operations by a switch, a touch panel, a touch pen, a keyboard, a mouse, and the like.

The notification unit 14 notifies the medical worker of the information processed by the control unit 10, such as the endoscopic image 4, the tracking destination virtual endoscopic image 6C, and the information related to the tracking of the viewpoint of the endoscope 2.

The notification of the information is to enable the medical worker to recognize the information. Therefore, for example, all of a form in which information is displayed on a display (not shown) which is an example of the display device, a form in which information is printed on a recording medium, such as paper, by an image forming apparatus (not shown), and a form in which information is notified by voice through a speaker (not shown) are examples of the notification of the information by the notification unit 14. In addition, a form in which information is transmitted through the I/F unit 12 is also an example of the notification of the information. The information processing apparatus 3 according to the embodiment of the present disclosure notifies of the information by the display of the information on the display and by voice notification.

The storage unit 15 stores, for example, the bronchial model 5 and various models such as the image conversion model 20, the trackability estimation model 21, the viewpoint difference estimation model 22, and the tracking result determination model 23. The storage unit 15 is an example of a storage device that retains stored information even in a case where power supplied to the storage unit 15 is cut off. For example, a semiconductor memory, such as a solid state drive (SSD), is used as the storage unit 15. However, a hard disk may be used.

Next, a process of the information processing apparatus 3 that tracks the viewpoint of the endoscope 2 will be described in detail.

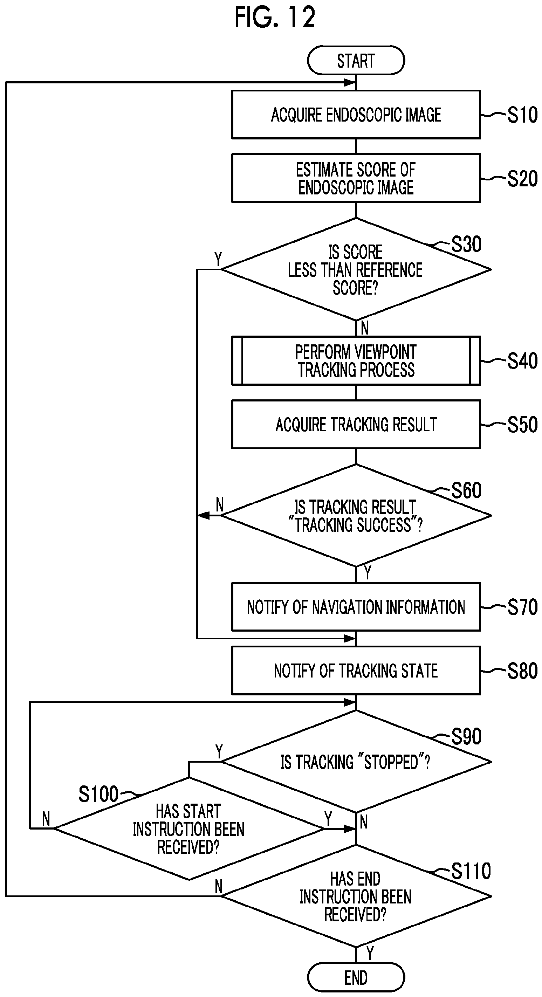

Fig. 12 is a flowchart showing an example of a flow of a tracking process executed by the information processing apparatus 3 in a case where an instruction to track the viewpoint of the endoscope 2 is received by the operation of the operation unit 13 by the medical worker. The CPU 10A of the information processing apparatus 3 reads the control program 11 from the ROM 10B and executes the tracking process. The control program 11 is an example of an information processing program according to the embodiment of the present disclosure.

In addition, it is assumed that the medical worker sets an initial viewpoint of the endoscope 2 in the bronchial model 5 in advance. The initial viewpoint of the endoscope 2 set by the medical worker may be a viewpoint different from the actual viewpoint of the endoscope 2. Alternatively, the CPU 10A may randomly set the initial viewpoint in the bronchial model 5.

In addition, it is assumed that the storage unit 15 stores the bronchial model 5 of the subject, the image conversion model 20, the trackability estimation model 21, the viewpoint difference estimation model 22, the tracking result determination model 23, and the reference score in advance.

First, in Step S10, the CPU 10A acquires the endoscopic image 4 from the endoscope 2 via the I/F unit 12. The endoscopic image 4 may be a color image or a monochrome image.

In Step S20, the CPU 10A inputs the endoscopic image 4 acquired by the process in Step S10 to the trackability estimation model 21 to estimate the score of the endoscopic image 4.

In Step S30, the CPU 10A determines whether or not the score of the endoscopic image 4 estimated by the process in Step S20 is less than the reference score.

In a case where the score of the endoscopic image 4 is equal to or greater than the reference score, the process proceeds to Step S40. In this case, the viewpoint of the endoscope 2 can be tracked. Therefore, in Step S40, the CPU 10A executes a viewpoint tracking process of specifying the viewpoint of the endoscope 2 that has captured the endoscopic image 4.

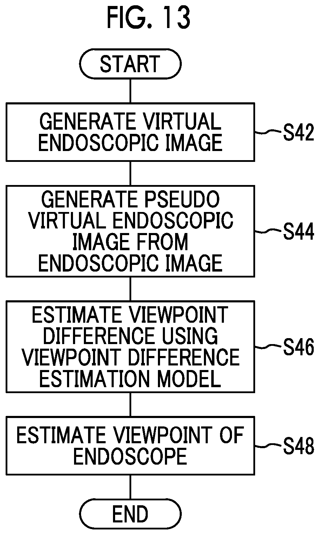

Fig. 13 is a flowchart showing an example of a flow of the viewpoint tracking process in Step S40.

In Step S42, the CPU 10A generates the virtual endoscopic image 6 in which the bronchial model 5 is viewed from the set viewpoint.

In Step S44, the CPU 10A inputs the endoscopic image 4 acquired by the process in Step S10 of Fig. 12 to the image conversion model 20 to generate the pseudo virtual endoscopic image 7 corresponding to the endoscopic image 4.

In Step S46, the CPU 10A inputs the virtual endoscopic image 6 generated by the process in Step S42 and the pseudo virtual endoscopic image 7 generated by the process in Step S44 to the viewpoint difference estimation model 22 to estimate the viewpoint difference between the endoscopic image 4 acquired by the process in Step S10 of Fig. 12 and the virtual endoscopic image 6 generated by the process in Step S42.

In Step S48, the CPU 10A reflects the viewpoint difference estimated by the process in Step S46 in the viewpoint set in the bronchial model 5 to estimate the viewpoint of the endoscope 2.

In this way, the viewpoint of the endoscope 2 is specified, and the viewpoint tracking process shown in Fig. 13 is ended.

After ending the viewpoint tracking process, in Step S50 of Fig. 12, the CPU 10A generates the tracking destination virtual endoscopic image 6C from the bronchial model 5 based on the tracked viewpoint of the endoscope 2 obtained by the viewpoint tracking process. In addition, the CPU 10A generates the tracking destination virtual depth image 17 corresponding to the position of the tracked viewpoint of the endoscope 2 from the bronchial model 5. Further, the CPU 10A inputs the endoscopic image 4 acquired by the process in Step S10, the tracking destination virtual endoscopic image 6C, and the tracking destination virtual depth image 17 to the tracking result determination model 23 to acquire the tracking result of the viewpoint of the endoscope 2.

In Step S60, in a case where the tracking result of the viewpoint of the endoscope 2 acquired by the process in Step S50 is "tracking success", the CPU 10A proceeds to Step S70.

In Step S70, the CPU 10A sets the viewpoint of the endoscope 2 specified by the process in Step S40 in the bronchial model 5 to update the viewpoint of the endoscope 2 in the bronchial model 5. The CPU 10A searches for a route from the updated viewpoint of the endoscope 2 to the target location using the bronchial model 5. For example, a known search method, such as a binary search method, can be used to search for the route to the target location.

The CPU 10A notifies the medical worker of the traveling direction of the endoscope 2, such as a "right direction" or a "downward direction", by voice through the speaker based on the search result of the route. In this way, the CPU 10A notifies the medical worker of the navigation information for the endoscope 2 to reach the target location and proceeds to Step S80.

On the other hand, in a case where it is determined in the determination process in Step S30 that the score of the endoscopic image 4 is less than the reference score, the CPU 10A proceeds to Step S80 without executing the processes in Steps S40 to S70. That is, the CPU 10A temporarily stops the tracking of the viewpoint of the endoscope 2 until the endoscopic image 4 having a score equal to or greater than the reference score is acquired.

In addition, in a case where it is determined in the determination process in Step S60 that the tracking result of the viewpoint of the endoscope 2 is "tracking failure", the CPU 10A proceeds to Step S80 without executing the process in Step S70. That is, in a case where the tracking of the viewpoint of the endoscope 2 has failed, the CPU 10A stops the tracking of the viewpoint of the endoscope 2.

In Step S80, the CPU 10A notifies the medical worker whether the tracking of the viewpoint of the endoscope 2 is in progress, is temporarily stopped, or is stopped. Specifically, in a case where the tracking result of the viewpoint of the endoscope 2 is "tracking success", the CPU 10A notifies the medical worker that the tracking is "in progress". In addition, in a case where the tracking result of the viewpoint of the endoscope 2 is "tracking failure", the CPU 10A notifies the medical worker that the tracking is "stopped". In addition, in a case where it is determined in the determination process in Step S30 that the score of the endoscopic image 4 is less than the reference score, the CPU 10A notifies the medical worker that the tracking is "temporarily stopped".

In a case where the tracking is "in progress", the CPU 10A generates the tracking destination virtual endoscopic image 6C in which the bronchial model 5 is viewed from the updated viewpoint of the endoscope 2 in the bronchial model 5 and displays, on the display, the generated tracking destination virtual endoscopic image 6C side by side with, for example, the endoscopic image 4 acquired by the process in Step S10. In this way, the tracking destination virtual endoscopic image 6C that follows a change in the viewpoint of the endoscope 2 is displayed.

In a case where the tracking is "stopped", the tracking of the viewpoint of the endoscope 2 is stopped. Therefore, it is not possible to generate the tracking destination virtual endoscopic image 6C unlike a case where the tracking is "in progress". Therefore, the CPU 10A displays, on the display, the tracking destination virtual endoscopic image 6C at the viewpoint at which the tracking has succeeded last before the tracking of the viewpoint of the endoscope 2 fails.

The fact that the tracking of the viewpoint of the endoscope 2 is stopped means that the medical worker has advanced the endoscope 2 beyond the allowable range that can be tracked by the information processing apparatus 3. Therefore, the medical worker needs to perform an operation of returning the position of the endoscope 2 to a position corresponding to the viewpoint at which the tracking has succeeded, in order to start tracking the viewpoint of the endoscope 2 again.

In the operation of the medical worker returning the endoscope 2, in a case where the tracking destination virtual endoscopic image 6C at the last viewpoint at which the tracking has succeeded is displayed on the display, it is easy for the medical worker to ascertain how far to return the endoscope 2, as compared to a case where the tracking destination virtual endoscopic image 6C is not displayed on the display.

Further, in a case where the virtual endoscopic image 6 including the characteristic shape of the bronchus, such as a bronchial bifurcation, is displayed on the display, it is easier for the medical worker to adjust the position of the endoscope 2, as compared to a case where the tracking destination virtual endoscopic image 6C including only the bronchial wall is displayed. Therefore, the CPU 10A may generate, from the bronchial model 5, the tracking destination virtual endoscopic image 6C including the bronchial bifurcation, which is an image generated from the viewpoint that has been successfully tracked at a point closer to the viewpoint that has been last successfully tracked before the tracking of the viewpoint of the endoscope 2 fails, and may display the tracking destination virtual endoscopic image 6C on the display.

As described above, the CPU 10A displays the tracking destination virtual endoscopic image 6C to be referred to by the medical worker to position the endoscope 2 on the display.

On the other hand, in a case where the tracking is "temporarily stopped", the CPU 10A displays, for example, the tracking destination virtual endoscopic image 6C at the last viewpoint that has been successfully tracked on the display until the tracking of the viewpoint of the endoscope 2 is resumed.

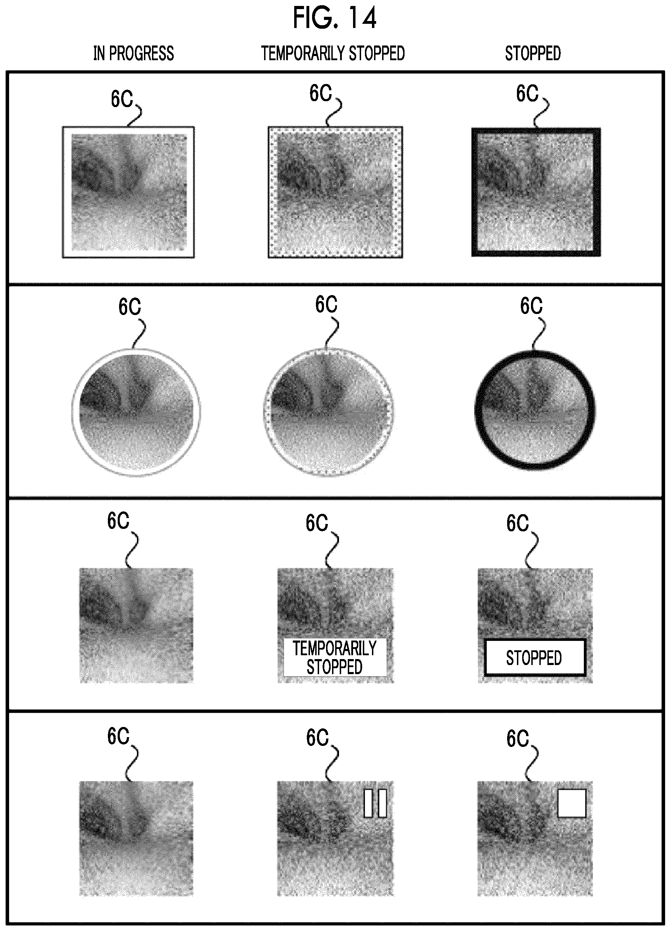

Fig. 14 is a diagram showing an example of a display form of the tracking destination virtual endoscopic image 6C for the medical worker. As shown in Fig. 14, the CPU 10A changes the display form of the tracking destination virtual endoscopic image 6C to notify the medical worker of the tracking state of the viewpoint of the endoscope 2.

Specifically, the CPU 10A changes the display form of a frame of the tracking destination virtual endoscopic image 6C according to the tracking state of the viewpoint of the endoscope 2. For example, the CPU 10A changes one or more of the color of the frame, the density of the color of the frame, and the thickness of the frame according to the tracking state of the viewpoint of the endoscope 2. In addition, there is no restriction on the shape of the tracking destination virtual endoscopic image 6C displayed on the display. Fig. 14 shows an example of the shape using a rectangle and a circle.

In addition, the CPU 10A may display letters or symbols indicating the tracking state to be superimposed on the tracking destination virtual endoscopic image 6C. In the example shown in Fig. 14, letters or symbols are not superimposed on the tracking destination virtual endoscopic image 6C in which the tracking of the viewpoint of the endoscope 2 is in progress. However, for example, a letter string "in progress" or a symbol (for example, a round symbol such as "o") indicating "in progress" may be superimposed on the tracking destination virtual endoscopic image 6C.

As described above, after the notification of the navigation information or the tracking state, in Step S90 of Fig. 12, the CPU 10A determines whether or not the tracking of the viewpoint of the endoscope 2 is "stopped". In a case where the tracking of the viewpoint of the endoscope 2 is "stopped", the CPU 10A proceeds to Step S100.

In a case where the tracking of the viewpoint of the endoscope 2 is "stopped", the medical worker positions the endoscope 2 while referring to the tracking destination virtual endoscopic image 6C displayed on the display. After the positioning of the endoscope 2 is completed, for example, the medical worker presses a tracking start button displayed on the display. Therefore, in Step S100, the CPU 10A determines whether or not an instruction to start the tracking of the viewpoint of the endoscope 2 notified by the pressing of the tracking start button has been received. In a case where the tracking start instruction has not been received, the CPU 10A proceeds to Step S90. That is, in a case where the tracking of the viewpoint of the endoscope 2 is "stopped", the CPU 10A stops the tracking process until the tracking start instruction is received.

In addition, the CPU 10A may notify the medical worker of the tracking state of the viewpoint of the endoscope 2 using the display form of the tracking start button. For example, in a case where the tracking of the viewpoint of the endoscope 2 is "in progress", the CPU 10A displays the tracking start button in a predetermined color (for example, green). In addition, in a case where the tracking of the viewpoint of the endoscope 2 is "temporarily stopped", the CPU 10A alternately displays (that is, blinks) the tracking start button in two different colors (for example, green and gray). Further, in a case where the tracking of the viewpoint of the endoscope 2 is "stopped", the CPU 10A displays the tracking start button in a color (for example, gray) different from that in a case where the tracking of the viewpoint of the endoscope 2 is "in progress".

On the other hand, in a case where it is determined in the determination process in Step S90 that the tracking of the viewpoint of the endoscope 2 is not "stopped" and in a case where it is determined in the determination process in Step S100 that the tracking start instruction has been received, the CPU 10A proceeds to Step S110.

In Step S110, the CPU 10A determines whether or not an instruction to end the tracking process has been received from the medical worker. In a case where the end instruction has not been received, the CPU 10A proceeds to Step S10 and acquires a new endoscopic image 4 from the endoscope 2. That is, the information processing apparatus 3 notifies the medical worker of the tracking state of the viewpoint of the endoscope 2 until the endoscope 2 receives the end instruction from the medical worker, determines the tracking result of the viewpoint of the endoscope 2 while tracking the viewpoint of the endoscope 2 according to the movement of the endoscope 2, and notifies the medical worker of the navigation information of the endoscope 2 in a case where the tracking has succeeded.

On the other hand, in a case where the end instruction has been received, the tracking process shown in Fig. 12 is ended.

As described above, according to the information processing apparatus 3 of the first embodiment, the tracking result of the viewpoint of the endoscope 2 is determined. In a case where the tracking of the viewpoint of the endoscope 2 has failed, the tracking of the viewpoint of the endoscope 2 is autonomously stopped. Therefore, the information processing apparatus 3 can suppress the notification of erroneous navigation information based on the viewpoint that is different from the actual viewpoint of the endoscope 2.

Further, in the tracking process shown in Fig. 12, in a case where the tracking of the viewpoint of the endoscope 2 is temporarily stopped and then the temporarily stopped state continues for a prescribed period of time or longer, a situation is considered in which a substance is attached to the lens of the endoscope 2 or a light of the endoscope 2 that illuminates the inside of the bronchus is out of order. Therefore, in a case where the temporarily stopped state continues for the prescribed period of time or longer in the tracking of the viewpoint of the endoscope 2, the CPU 10A may change the tracking state of the viewpoint of the endoscope 2 to the "stopped" state. The prescribed period of time for changing the tracking state to the "stopped" state is set by, for example, the medical worker and is stored in the storage unit 15 in advance.

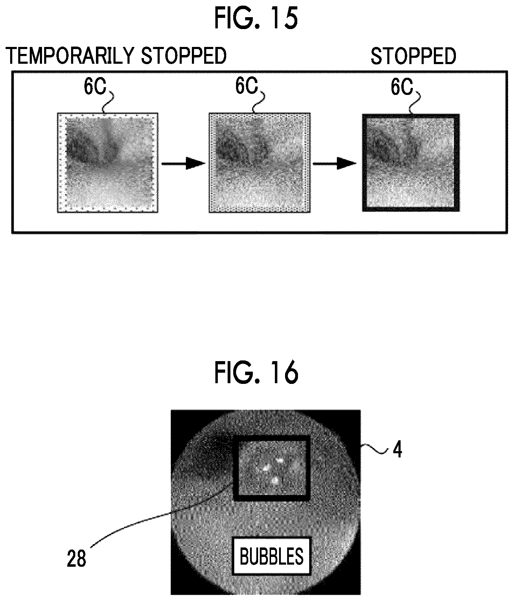

Fig. 15 is a diagram showing an example of the display form of the tracking destination virtual endoscopic image 6C in a case where the temporarily stopped state continues for the prescribed period of time or longer in the tracking of the viewpoint of the endoscope 2 and the tracking state of the viewpoint of the endoscope 2 is changed to the "stopped" state. As shown in Fig. 15, the CPU 10A changes the display form of the frame of the tracking destination virtual endoscopic image 6C according to the length of the elapsed time from the temporary stop of the tracking of the viewpoint of the endoscope 2 such that the tracking state at the current time can be known. In a case where the elapsed time from the temporary stop is equal to or longer than the prescribed period of time, the CPU 10A fixes the display form of the frame to the display form indicating the tracking state of "stopped". The display form of the frame until the tracking state is changed from the "temporarily stopped" state to the "stopped" state may be changed continuously or stepwise.

In addition, the fact that the tracking of the viewpoint of the endoscope 2 is temporarily stopped means that the endoscopic image 4 includes the unknown structure region that makes it difficult to track the viewpoint of the endoscope 2. Therefore, in a case where the tracking of the viewpoint of the endoscope is "temporarily stopped", the CPU 10A may notify of the reason for temporarily stopping the tracking of the viewpoint of the endoscope 2.

Fig. 16 is a diagram showing an example of the display of the unknown structure region in the endoscopic image 4. As shown in Fig. 16, for example, the CPU 10A surrounds the unknown structure region with a display frame 28 to notify the medical worker of the unknown structure region. In addition, the CPU 10A may display the type of the unknown structure region together with the display of the position of the unknown structure region by the display frame 28. In the example shown in Fig. 16, the unknown structure region surrounded by the display frame 28 is displayed as "bubbles".

For example, an unknown structure region detection model is used to detect the unknown structure region from the endoscopic image 4.

The unknown structure region detection model is a model that detects the unknown structure region from the endoscopic image 4 and outputs positional information of the unknown structure region and the type of the unknown structure region in the endoscopic image 4 in a case where the endoscopic image 4 is input. The unknown structure region detection model is generated by machine learning using, for example, learning data in which the endoscopic image 4 is input data and the positional information of the unknown structure region and the type of the unknown structure region are training data in the endoscopic image 4. For example, the medical worker associates the endoscopic image 4 with the positional information of the unknown structure region and the type of the unknown structure region in the endoscopic image 4 in the learning data.

The medical worker who has been notified of the presence of the unknown structure region may examine the cause of the detection of the unknown structure region to suppress the occurrence of the unknown structure region. In a case where the unknown structure region is caused by the adhesion of bubbles or foreign substances, it is possible to remove the unknown structure region using, for example, forceps or water supply. In addition, in a case where the unknown structure region is caused by the reflection of illumination light, for example, the unknown structure region can be removed by reducing the amount of illumination light.

The information processing apparatus 3 can track the viewpoint of the endoscope 2 even in a case where the endoscopic image 4 includes the unknown structure region. However, as the number of unknown structure regions is smaller, the accuracy of tracking is more improved. Therefore, the information processing apparatus 3 notifies the medical worker of the position of the detected unknown structure region to support the operation of the medical worker removing the cause of the occurrence of the unknown structure region.

In addition, as the area of the unknown structure region is smaller, the influence on the tracking of the viewpoint of the endoscope 2 is smaller. In a case where the position of the unknown structure region is notified regardless of the area of the detected unknown structure region, even though a treatment to remove the cause of the occurrence of the unknown structure region is performed, this does not contribute to improving the accuracy of estimation of the viewpoint of the endoscope 2 and may impose an extra burden on the medical worker. Therefore, in a case where the proportion of the unknown structure region to the endoscopic image 4 is equal to or greater than a prescribed proportion, the CPU 10A may be configured to notify of the position of the unknown structure region in the endoscopic image 4.

In addition, in the tracking process shown in Fig. 12, the CPU 10A estimates whether or not the viewpoint of the endoscope 2 can be tracked by the determination process in Step S30 and then performs the viewpoint tracking process to determine the tracking result of the viewpoint of the endoscope 2. However, in a case where the tracking state of the viewpoint of the endoscope 2 can be correctly identified, there is no restriction on the order of the processes in the tracking process. After the viewpoint tracking process is executed, it may be estimated whether or not the viewpoint of the endoscope 2 can be tracked.

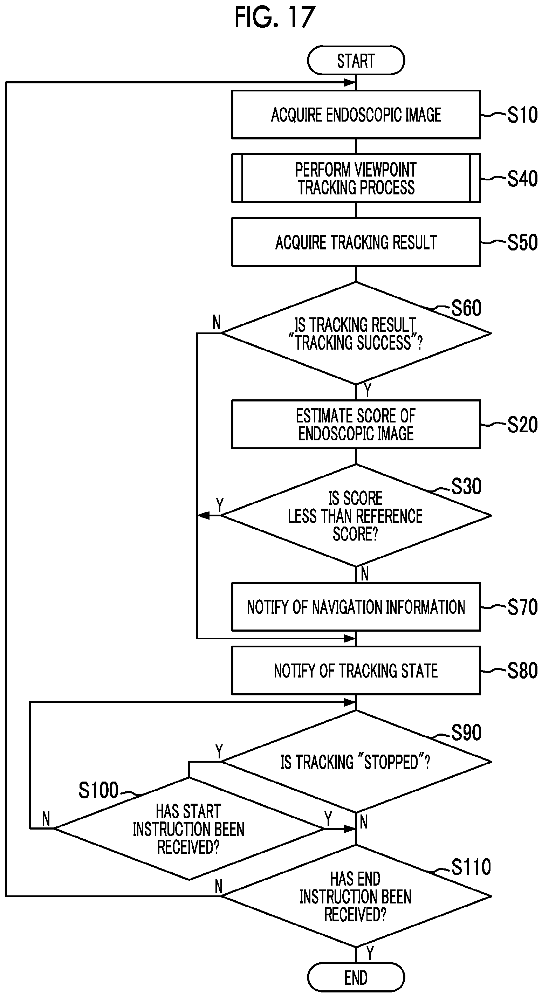

Fig. 17 is a flowchart showing a modification example of the flow of the tracking process executed by the information processing apparatus 3 in a case where an instruction to start the tracking of the viewpoint of the endoscope 2 has been received by the operation of the medical worker through the operation unit 13.

The flowchart shown in Fig. 17 differs from the flowchart of the tracking process shown in Fig. 12 in that the process in Steps S40 to S60 is executed after the process in Step S10 is executed and the process in Steps S20 and S30 is executed in a case where the tracking result of the viewpoint of the endoscope 2 by the determination process in Step S60 is "tracking success".

That is, the CPU 10A first executes the viewpoint tracking process using the endoscopic image 4 acquired from the endoscope 2. In a case where the tracking result of the viewpoint of the endoscope 2 is "tracking failure", the CPU 10A sets the tracking state to the "stopped" state. On the other hand, in a case where the tracking result of the viewpoint of the endoscope 2 is "tracking success", the CPU 10A estimates the trackability of the endoscope 2 in the endoscopic image 4 used in the viewpoint tracking process. In a case where the score of the endoscopic image 4 is equal to or greater than the reference score, the CPU 10A sets the tracking state to the "in progress" state. In a case where the score of the endoscopic image 4 is less than the reference score, the CPU 10A sets the tracking state to the "temporarily stopped" state.

As described above, in a case where the tracking state of the viewpoint of the endoscope 2 can be correctly identified, there is no restriction on the order of the processes in the tracking process. Therefore, for example, in a case where the estimation of the trackability of the endoscope 2 in Step S20 is performed in parallel with the process in Steps S40 to S60 shown in Fig. 17, it is possible to shorten the time from the acquisition of the endoscopic image 4 to the notification of the navigation information, as compared to a case where the processes are sequentially executed.

### Modification Example of Tracking Result Determination Model 23

The example in which the tracking result of the viewpoint of the endoscope 2 is determined using the tracking result determination model 23 generated by the machine learning using the determination learning data has been described above. However, the tracking result determination model 23 can also be constructed using a method other than the machine learning.

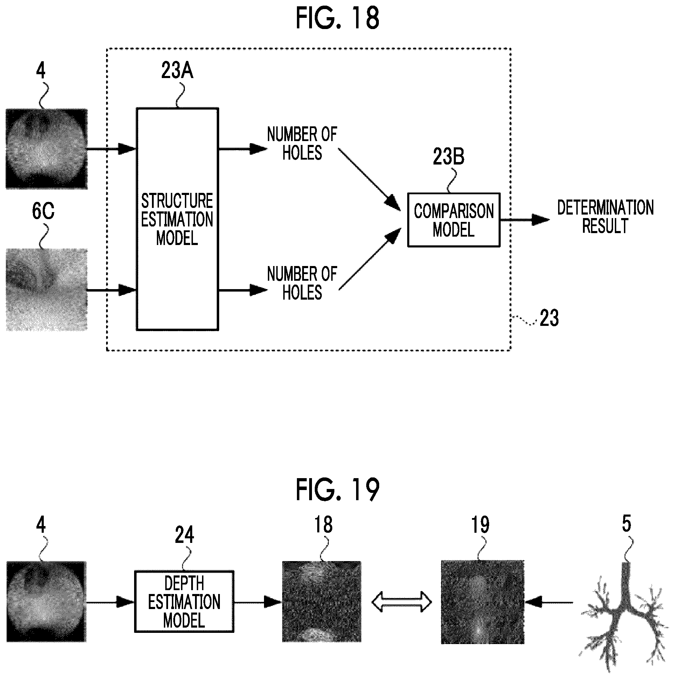

Fig. 18 is a diagram showing a modification example of the tracking result determination model 23. The tracking result determination model 23 according to the modification example includes a structure estimation model 23A and a comparison model 23B.

The structure estimation model 23A estimates structure information indicating a structure of a living body from an input image. The structure information estimated by the structure estimation model 23A is not restricted. For example, at least one of the number of holes, the texture of the bronchus, or the shape of the tumor can be estimated. For example, it is assumed that the number of holes formed by the bronchi is estimated. For example, a known image processing method based on shape recognition or feature amount extraction is used to estimate the structure of the living body from the image.

The comparison model 23B compares two structure information items estimated from different images by the structure estimation model 23A, determines that the two images show the same location in a case where the structure information items are similar, and determines that the two images show different locations in a case where the structure information items are not similar. There is no restriction on a method for comparing the structure information items in the comparison model 23B, and a known comparison method is used.

In a case where the endoscopic image 4 and the tracking destination virtual endoscopic image 6C are input to the tracking result determination model 23 according to this modification example, the number of holes included in the endoscopic image 4 is compared with the number of holes included in the tracking destination virtual endoscopic image 6C. In a case where the numbers of holes are equal to each other, the tracking result determination model 23 according to the modification example outputs a determination result indicating that the endoscopic image 4 and the tracking destination virtual endoscopic image 6C show the same location. In a case where the numbers of holes are different from each other, the tracking result determination model 23 according to the modification example outputs a determination result indicating that the endoscopic image 4 and the tracking destination virtual endoscopic image 6C show different locations.

The fact that the input endoscopic image 4 and the input tracking destination virtual endoscopic image 6C show the same location means that the bronchial model 5 is viewed at the tracked viewpoint which is the same as the viewpoint of the endoscope 2. Therefore, the tracking result of the viewpoint of the endoscope 2 is "tracking success". On the other hand, the fact that the input endoscopic image 4 and the input tracking destination virtual endoscopic image 6C show different locations means that the bronchial model 5 is viewed at the tracked viewpoint which is different from the viewpoint of the endoscope 2. Therefore, the tracking result of the viewpoint of the endoscope 2 is "tracking failure".

As described above, the information processing apparatus 3 may compare the structure information items obtained from the endoscopic image 4 and the tracking destination virtual endoscopic image 6C, using a known image processing method or a known comparison method, to determine the tracking result of the viewpoint of the endoscope 2.

Further, in Fig. 18, the example in which the tracking result of the viewpoint of the endoscope 2 is determined from the endoscopic image 4 and the tracking destination virtual endoscopic image 6C has been described. However, the depth image corresponding to the endoscopic image 4 and the tracking destination virtual depth image 17 may be input to the tracking result determination model 23 according to the modification example to determine the tracking result of the viewpoint of the endoscope 2. In addition, the endoscopic image 4 and the tracking destination virtual depth image 17 may be input to the tracking result determination model 23 according to the modification example to determine the tracking result of the viewpoint of the endoscope 2, or the endoscopic image 4, the tracking destination virtual endoscopic image 6C, and the tracking destination virtual depth image 17 may be input to the tracking result determination model 23 according to the modification example to determine the tracking result of the viewpoint of the endoscope 2. In this configuration, in a case where the structure information obtained from the endoscopic image 4, the structure information obtained from the tracking destination virtual endoscopic image 6C, and the structure information obtained from the tracking destination virtual depth image 17 are similar to each other, the comparison model 23B may determine that the images show the same location. In a case where the structure information items are not similar to each other, the comparison model 23B may determine that the images show different locations.

Further, a plurality of images obtained from the endoscope 2 and a plurality of images obtained from the bronchial model 5, such as the endoscopic image 4 and the tracking destination virtual endoscopic image 6C, and the depth image corresponding to the endoscopic image 4 and the tracking destination virtual depth image 17, may be input to the tracking result determination model 23 according to the modification example to determine the tracking result of the viewpoint of the endoscope 2.

Furthermore, instead of the tracking destination virtual endoscopic image 6C shown in Fig. 18, a partial bronchial model viewed from the tracked viewpoint may be input to the tracking result determination model 23 according to the modification example. In this case, the structure estimation model 23A estimates the number of holes which are visible in a case where the partial bronchial model is viewed from the tracked viewpoint for the input partial bronchial model.

### Second Embodiment

In the first embodiment, the example has been described in which the viewpoint difference is estimated using the viewpoint difference estimation model 22 generated by machine learning and the viewpoint of the endoscope 2 is tracked based on the estimated viewpoint difference.

However, a method for tracking the viewpoint of the endoscope 2 is not limited to the method using the viewpoint difference estimation model 22 generated by machine learning. Any method may be used as long as it can track the viewpoint of the endoscope 2.

In the second embodiment, the information processing apparatus 3 that tracks the viewpoint of the endoscope 2 based on a similarity between images will be described.

An example of a functional configuration of the information processing apparatus 3 according to the second embodiment is the same as the example of the functional configuration of the information processing apparatus 3 shown in Fig. 1, and the information processing apparatus 3 is configured using the computer shown in Fig. 11.

The information processing apparatus 3 estimates the viewpoint of the endoscope 2 from a similarity between a depth image (hereinafter, referred to as an "endoscopic depth image 18") corresponding to the endoscopic image 4 and a depth image generated using the bronchial model 5. The similarity is represented by, for example, a numerical value. For example, it is assumed that, as the numerical value of the similarity is larger, the similarity between the images is higher.

Therefore, the information processing apparatus 3 generates the endoscopic depth image 18 from the endoscopic image 4, which will be described below. In addition, the information processing apparatus 3 generates a depth image (hereinafter, referred to as a "virtual depth image 19") at each position inside the bronchial model 5.

The information processing apparatus 3 calculates the similarity between each generated virtual depth image 19 and the endoscopic depth image 18 and specifies a virtual depth image 19 most similar to the endoscopic depth image 18 from the virtual depth images 19. In addition, the information processing apparatus 3 searches for a viewpoint at which an image having the highest similarity to the endoscopic image 4, which is the generation source of the endoscopic depth image 18, is obtained at the position indicated by the specified virtual depth image 19 and estimates the viewpoint of the endoscope 2. That is, the viewpoint at which the image having the highest similarity to the endoscopic image 4 is obtained at the position indicated by the virtual depth image 19 most similar to the endoscopic depth image 18 is the viewpoint of the endoscope 2.

In addition, for example, a known image recognition method that compares the feature amounts of images to calculate the similarity can be applied to calculate the similarity between the endoscopic depth image 18 and the virtual depth image 19 (simply referred to as a "similarity between images"). Further, a similarity estimation model which is a model generated by machine learning and outputs the similarity between the images may be used to calculate the similarity between the images.

Fig. 19 is a diagram showing an example of a method for estimating the similarity between the images in the information processing apparatus 3 according to the second embodiment. It is assumed that the generation unit 3E generates the virtual depth image 19 at each position of the bronchial model 5 before the similarity is estimated.

For example, the tracking unit 3F inputs the endoscopic image 4 to a depth estimation model 24 to convert the endoscopic image 4 into the endoscopic depth image 18. The depth estimation model 24 is, for example, a model generated in advance using a GAN that has been trained to output the endoscopic depth image 18 from the endoscopic image 4 and is stored in advance in the storage unit 3D.

The tracking unit 3F calculates the similarity between each virtual depth image 19 generated by the generation unit 3E and the endoscopic depth image 18 to estimate the viewpoint of the endoscope 2 and tracks the viewpoint of the endoscope 2.

Next, a process of the information processing apparatus 3 that tracks the viewpoint of the endoscope 2 based on the similarity between the images will be described in detail.

A tracking process executed by the information processing apparatus 3 according to the second embodiment follows the same flowchart as the tracking process shown in Fig. 12 or Fig. 17. However, the tracking processes are different in the viewpoint tracking process in Step S40 of Figs. 12 and 17. Therefore, hereinafter, the viewpoint tracking process according to the second embodiment in Step S40 of Figs. 12 and 17 will be described.

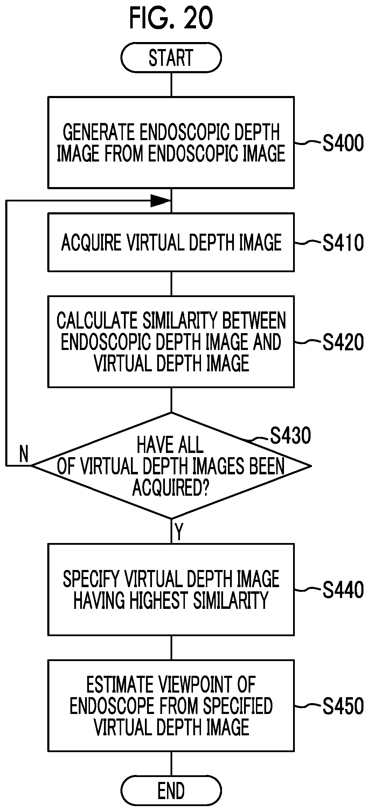

Fig. 20 is a flowchart showing an example of a flow of the viewpoint tracking process according to the second embodiment. It is assumed that the storage unit 15 stores the bronchial model 5 of the subject, the virtual depth image 19 at each position inside the bronchial model 5 of the subject, the trackability estimation model 21, the depth estimation model 24, the tracking result determination model 23, and the reference score in advance.

In Step S400, the CPU 10A inputs the endoscopic image 4 acquired by the process in Step S10 shown in Fig. 12 or Fig. 17 to the depth estimation model 24 to generate the endoscopic depth image 18.

In Step S410, the CPU 10A acquires any one virtual depth image 19 from the plurality of virtual depth images 19 stored in the storage unit 15.

In Step S420, the CPU 10A calculates the similarity between the endoscopic depth image 18 generated by the process in Step S400 and the virtual depth image 19 acquired by the process in Step S410 and stores the calculated similarity in the RAM 10C in association with the virtual depth image 19 used to calculate the similarity.

In Step S430, the CPU 10A determines whether or not all of the virtual depth images 19 stored in the storage unit 15 have been acquired. In a case where all of the virtual depth images 19 have not been acquired, the CPU 10A proceeds to Step S410 and acquires one virtual depth image 19 that has not been acquired. That is, the CPU 10A repeats the processes in Steps S410 to S430 until it is determined in the determination process in Step S430 that all of the virtual depth images 19 have been acquired and associates the similarity between the endoscopic depth image 18 and each virtual depth image 19 with each virtual depth image 19.

On the other hand, in a case where it is determined in the determination process in Step S430 that all of the virtual depth images 19 have been acquired, the CPU 10A proceeds to Step S440.

In Step S440, the CPU 10A specifies a virtual depth image 19, with which the highest similarity is associated, among all of the virtual depth images 19 as the virtual depth image 19 that is most similar to the endoscopic depth image 18 with reference to the similarities associated with each virtual depth image 19 stored in the RAM 10C.

In Step S450, the CPU 10A searches for a viewpoint at which an image having the highest similarity to the endoscopic image 4, which is the generation source of the endoscopic depth image 18, is obtained at the position of the bronchial model 5 indicated by the virtual depth image 19 specified by the process in Step S440 to estimate the viewpoint of the endoscope 2. In this way, the viewpoint tracking process according to the second embodiment shown in Fig. 20 is ended.

According to the information processing apparatus 3 of the second embodiment, the viewpoint of the endoscope 2 is estimated based on the similarity between the endoscopic depth image 18 and the virtual depth image 19. Then, the viewpoint of the endoscope 2 is tracked. As described above, a method for tracking the viewpoint of the endoscope 2 in the information processing apparatus 3 is not limited to the method using the viewpoint difference estimation model 22, and other methods may be used. In other words, the viewpoint tracking process of the endoscope 2 in the information processing apparatus 3 can be executed without being affected by the method for tracking the viewpoint of the endoscope 2.

One form of the information processing system 1 has been described above using the embodiment. However, the disclosed form of the information processing system 1 is an example. The form of the information processing system 1 is not limited to the range described in the embodiment. Various modifications and improvements can be added to the embodiments without departing from the gist of the present disclosure, and the embodiments to which the modifications or improvements are added are also included in the technical scope of the present disclosure.

For example, the internal processing order in the flowchart of each tracking process shown in Figs. 12 and 17 and the internal processing order in the flowchart of each viewpoint tracking process shown in Figs. 13 and 20 may be changed without departing from the gist of the present disclosure.

In each of the above-described embodiments, the form in which each tracking process is implemented by software processing has been described as an example. However, the same processes as those in the flowcharts of each tracking process may be performed by hardware. In this case, the processing speed can be increased as compared to a case where each tracking process is implemented by the software processing.

In the above-described embodiments, the term "processor" refers to hardware in a broad sense. Examples of the processor include general processors (for example, the CPU 10A) and dedicated processors (for example, a graphics processing unit: GPU, an application specific integrated circuit: ASIC, a field programmable gate array: FPGA, and a programmable logic device).

Further, the operation of the processor in each of the above-described embodiments may be performed not only by one processor but also by cooperation of a plurality of processors provided at physically separated positions. In addition, the order of the operations of the processor is not limited to only the order described in the embodiments and may be changed as appropriate.

In each of the above-described embodiments, the example in which the control program 11 is stored in the ROM 10B has been described. However, the storage destination of the control program 11 is not limited to the ROM 10B. The control program 11 can also be provided in a form in which the control program 11 is recorded in a computer-readable storage medium.

For example, the control program 11 may be provided in a form in which the control program 11 is recorded in an optical disk such as a compact disk read only memory (CD-ROM), a digital versatile disk read only memory (DVD-ROM), or a Blu-ray disc. In addition, the control program 11 may be provided in a form in which the control program 11 is recorded in a portable semiconductor memory such as a universal serial bus (USB) memory or a memory card. The ROM 10B, the CD-ROM, the DVD-ROM, the Blu-ray disc, the USB, and the memory card are examples of a non-transitory storage medium.

Further, the control unit 10 may download the control program 11 from an external apparatus connected to the communication line via the I/F unit 12 and store the downloaded control program 11 in the ROM 10B of the control unit 10.

For the above-described embodiments, the following supplementary notes are further disclosed.

### Supplementary Note 1

An information processing apparatus comprising:
an estimation unit that estimates whether or not a viewpoint of an endoscope that has captured an endoscopic image of a living body is trackable from the endoscopic image;
a tracking unit that tracks the viewpoint of the endoscope using the endoscopic image and an image generated from a three-dimensional model of the living body in a case where the estimation unit estimates that the viewpoint of the endoscope is trackable;
a determination unit that determines a tracking result of the viewpoint of the endoscope by the tracking unit using the endoscopic image and a three-dimensional model image generated from the three-dimensional model based on the tracked viewpoint of the endoscope; and
a controller that performs control to stop the tracking of the viewpoint of the endoscope until a tracking start instruction is notified in a case where the determination unit determines that the tracking has failed.

### Supplementary Note 2

The information processing apparatus according to Supplementary Note 1,
wherein the three-dimensional model image is at least one of a tracking destination virtual endoscopic image of the living body, which is an image in a case where the three-dimensional model is viewed from the tracked viewpoint of the endoscope, or a tracking destination virtual depth image, which is a depth image of the three-dimensional model corresponding to a position where the tracking destination virtual endoscopic image has been obtained, and
the determination unit determines the tracking result of the viewpoint of the endoscope using a determination model that has been subjected to machine learning in advance to output whether or not the tracking of the viewpoint of the endoscope has succeeded from the tracking destination virtual endoscopic image, the tracking destination virtual depth image, and the endoscopic image.

### Supplementary Note 3

The information processing apparatus according to Supplementary Note 1,
wherein the three-dimensional model image is at least one of a tracking destination virtual endoscopic image of the living body, which is an image in a case where the three-dimensional model is viewed from the tracked viewpoint of the endoscope, or a tracking destination virtual depth image, which is a depth image of the three-dimensional model corresponding to a position where the tracking destination virtual endoscopic image has been obtained, and
the determination unit compares structure information items, which indicate a structure of the living body and have been obtained from the endoscopic image and at least one of the tracking destination virtual endoscopic image or the tracking destination virtual depth image, to determine the tracking result of the viewpoint of the endoscope.

### Supplementary Note 4

The information processing apparatus according to any one of Supplementary Notes 1 to 3,
wherein, in a case where the estimation unit estimates that the viewpoint of the endoscope is not trackable from the endoscopic image, the estimation unit temporarily stops the tracking of the viewpoint of the endoscope until the endoscopic image, from which it is estimated that the viewpoint of the endoscope is trackable, is acquired.

### Supplementary Note 5

The information processing apparatus according to any one of Supplementary Notes 1 to 4,
wherein, in a case where the determination unit determines that the tracking has failed, the controller performs control to display, on a display device, a virtual endoscopic image that is referred to by a medical worker to position the endoscope and that is obtained in a case where the three-dimensional model is viewed from a viewpoint at which the tracking has succeeded.

### Supplementary Note 6

The information processing apparatus according to Supplementary Note 5,
wherein the controller performs control to display, on the display device, the virtual endoscopic image at a viewpoint at which the tracking of the viewpoint of the endoscope has succeeded last before the tracking fails.

### Supplementary Note 7

The information processing apparatus according to Supplementary Note 5,
wherein the controller performs control to display, on the display device, the virtual endoscopic image including a bronchial bifurcation located closer to a viewpoint at which the tracking of the viewpoint of the endoscope has succeeded last before the tracking fails.

### Supplementary Note 8

The information processing apparatus according to any one of Supplementary Notes 1 to 7,
wherein the controller performs control to notify whether the tracking of the viewpoint of the endoscope is in progress, is temporarily stopped, or is stopped.

### Supplementary Note 9

The information processing apparatus according to Supplementary Note 8,
wherein the controller changes a display form of the three-dimensional model image to notify of a tracking state of the viewpoint of the endoscope.

### Supplementary Note 10

The information processing apparatus according to Supplementary Note 9,
where, in a case where the tracking of the viewpoint of the endoscope is temporarily stopped, the controller performs control to notify of a reason for temporarily stopping the tracking of the viewpoint of the endoscope.

### Supplementary Note 11

An information processing program causing a computer to execute a process comprising:
estimating whether or not a viewpoint of an endoscope that has captured an endoscopic image of a living body is trackable from the endoscopic image;
tracking the viewpoint of the endoscope using the endoscopic image and an image generated from a three-dimensional model of the living body in a case where it is estimated that the viewpoint of the endoscope is trackable;
determining a tracking result of the viewpoint of the endoscope using the endoscopic image and a three-dimensional model image generated from the three-dimensional model based on the tracked viewpoint of the endoscope; and
stopping the tracking of the viewpoint of the endoscope until a tracking start instruction is notified in a case where it is determined that the tracking has failed.

## Claims

1. An information processing apparatus (3) comprising:
an estimation unit (3B) that estimates whether or not a viewpoint of an endoscope (2) that has captured an endoscopic image (4) of a living body is trackable from the endoscopic image (4);
a tracking unit (3F) that tracks the viewpoint of the endoscope (2) using the endoscopic image (4) and a three-dimensional model (5) of the living body in a case where the estimation unit (3B) estimates that the viewpoint of the endoscope (2) is trackable;
a determination unit (3G) that determines a tracking result of the viewpoint of the endoscope (2) by the tracking unit (3F) using the three-dimensional model (5) and the endoscopic image (4) based on the tracked viewpoint of the endoscope (2); and
a controller (3H) that performs control to stop the tracking of the viewpoint of the endoscope (2) until a tracking start instruction is notified in a case where the determination unit (3G) determines that the tracking has failed.

2. The information processing apparatus (3) according to claim 1,
wherein a three-dimensional model image (6C, 17) generated from the three dimensional model (5) is at least one of a tracking destination virtual endoscopic image (6C) of the living body, which is an image in a case where the three-dimensional model (5) is viewed from the tracked viewpoint of the endoscope (2), or a tracking destination virtual depth image (17), which is a depth image of the three-dimensional model (5) corresponding to a position where the tracking destination virtual endoscopic image (6C) has been obtained, and
the determination unit (3G) determines the tracking result of the viewpoint of the endoscope (2) using a determination model that has been subjected to machine learning in advance to output whether or not the tracking of the viewpoint of the endoscope (2) has succeeded from the tracking destination virtual endoscopic image (6C), the tracking destination virtual depth image (17), and the endoscopic image.

3. The information processing apparatus (3) according to claim 1,
wherein a three-dimensional model image (6C, 17) generated from the three dimensional model (5) is at least one of a tracking destination virtual endoscopic image (6C) of the living body, which is an image in a case where the three-dimensional model (5) is viewed from the tracked viewpoint of the endoscope (2), or a tracking destination virtual depth image (17), which is a depth image of the three-dimensional model (5) corresponding to a position where the tracking destination virtual endoscopic image (17) has been obtained, and
the determination unit (3G) compares structure information items, which indicate a structure of the living body and have been obtained from the endoscopic image (4) and at least one of the tracking destination virtual endoscopic image (6C) or the tracking destination virtual depth image (17), to determine the tracking result of the viewpoint of the endoscope (2).

4. The information processing apparatus (3) according to any one of claims 1 to 3,
wherein, in a case where the estimation unit (3B) estimates that the viewpoint of the endoscope (2) is not trackable from the endoscopic image (4), the estimation unit temporarily stops the tracking of the viewpoint of the endoscope (2) until the endoscopic image (4), from which it is estimated that the viewpoint of the endoscope (2) is trackable, is acquired.

5. The information processing apparatus (3) according to any one of claims 1 to 4,
wherein, in a case where the determination unit (3G) determines that the tracking has failed, the controller (3H) performs control to display, on a display device, a virtual endoscopic image (6, 6A, 6B) that is referred to by a medical worker to position the endoscope (2) and that is obtained in a case where the three-dimensional model (5) is viewed from a viewpoint at which the tracking has succeeded.

6. The information processing apparatus (3) according to claim 5,
wherein the controller (3H) performs control to display, on the display device, the virtual endoscopic image (6, 6A, 6B) at a viewpoint at which the tracking of the viewpoint of the endoscope (2) has succeeded last before the tracking fails.

7. The information processing apparatus (3) according to claim 5 or 6,
wherein the controller (3H) performs control to display, on the display device, the virtual endoscopic image (6, 6A, 6B) including a bronchial bifurcation located closer to a viewpoint at which the tracking of the viewpoint of the endoscope (2) has succeeded last before the tracking fails.

8. The information processing apparatus (3) according to any one of claims 4 to 7,
wherein the controller (3H) performs control to notify whether the tracking of the viewpoint of the endoscope (2) is in progress, is temporarily stopped, or is stopped.

9. The information processing apparatus (3) according to claim 8,
wherein the controller (3H) changes a display form of the three-dimensional model image (6C, 17) generated from the three dimensional model (5) to notify of a tracking state of the viewpoint of the endoscope (2).

10. The information processing apparatus (3) according to claim 9,
wherein, in a case where the tracking of the viewpoint of the endoscope (2) is temporarily stopped, the controller (3H) performs control to notify of a reason for temporarily stopping the tracking of the viewpoint of the endoscope (2).

11. A storage medium (19B) storing an information processing program (11) executable by a computer (10) to execute a process comprising:
estimating whether or not a viewpoint of an endoscope (2) that has captured an endoscopic image (4) of a living body is trackable from the endoscopic image (4);
tracking the viewpoint of the endoscope using the endoscopic image (4) and a three-dimensional model (5) of the living body in a case where it is estimated that the viewpoint of the endoscope (2) is trackable;
determining a tracking result of the viewpoint of the endoscope (2) using the three-dimensional model (5) and the endoscopic image (4) based on the tracked viewpoint of the endoscope (2); and
stopping the tracking of the viewpoint of the endoscope (2) until a tracking start instruction is notified in a case where it is determined that the tracking has failed.
